# EUROPEAN PATENT APPLICATION

(11) **EP 4 083 211 A1**
(43) Date of publication of application: **02.11.2022**
(21) Application number: 20907503.5
(22) Date of filing: 24.12.2020
(51) Int. Cl.: C12N 15/13, A61K 39/395, A61P 35/00, A61P 35/02, C07K 16/28, C07K 16/46, C12N 15/62

(54) **ANTI-CDCP1 ANTIBODY**

(30) Priority: 27.12.2019 JP 2019238928
(71) Applicant: Chiome Bioscience, Inc, Tokyo 151-0071 (JP)
(72) Inventor: HASHIMOTO Shuichi, Tokyo 151-0071 (JP); NAKAMURA Koji, Tokyo 151-0071 (JP); SANO Hitomi, Tokyo 151-0071 (JP); YOSHIOKA Akiko, Tokyo 151-0071 (JP); TAKESUE Aki, Tokyo 151-0071 (JP)
(74) Representative: J A Kemp LLP
(86) International application number: PCT/JP2020/048347
(87) International publication number: WO 2021/132427

(57) **Abstract**

The present invention is intended to provide: an anti-hCDCP1 antibody, which can be used as an active ingredient of anticancer agents having few side effects, etc.; and the aforementioned anti-hCDCP1 antibody that is formulated into ADC. Specifically, the present invention relates to an antibody that binds to human CDCP1 (CUB domain-containing protein 1), which has low binding property to human CD34-positive cells, or an antigen-binding fragment thereof. A representative example of the present antibody may be an antibody having heavy chain CDR1 comprising the amino acid sequence as set forth in SEQ ID NO: 25, heavy chain CDR2 comprising the amino acid sequence as set forth in SEQ ID NO: 26, heavy chain CDR3 comprising the amino acid sequence as set forth in SEQ ID NO: 27, light chain CDR1 comprising the amino acid sequence as set forth in SEQ ID NO: 29, light chain CDR2 comprising the amino acid sequence as set forth in SEQ ID NO: 30, and light chain CDR3 comprising the amino acid sequence as set forth in SEQ ID NO: 31.

## Description

### Technical Field

The present invention relates to an anti-CDCP1 antibody. More specifically, the present invention relates to: an anti-human CDCP1 antibody having low binding property to the bone marrow hematopoietic stem cells of a healthy human subject, and a fragment thereof; and use of them.

### Background Art

Human CDCP1 (cub domain containing protein 1) (hereinafter also referred to as "hCDCP1") is a type I transmembrane protein consisting of 836 amino acids over its entire length and having three CUB domains (complement C1r/C1s, Uegf, and Bmp1 domains) (see Non Patent Literature 1). The CUB domains present in a large number of proteins, including BMP1, C1r, proteins having protease activity, such as TMPRSS7, and proteins associated with intercellular interaction, such as LRP3, NRP1 and TLL2, but unified understanding regarding its molecular functions is insufficient. A large number of N-glycosylation sites are present on the N-terminal side of an extracellular domain, and a correlation is found between the degree of glycosylation and the metastatic state of cancer cells (see Non Patent Literature 2). hCDCP1 is expressed in various cells, but its soluble ligand is unknown. On the other hand, it is suggested that hCDCP1 should interact with various membrane proteins such as EGFR (see Non Patent Literature 3). The hCDCP1 protein shows a band size of 135 kDa in electrophoresis. When the amino acids at positions 368/369 or 369/370 between the CUB1 domain and the CUB2 domain of the hCDCP1 protein are cleaved by serine protease, 70 kDa fragments are generated (see Non Patent Literature 1).

In hCDCP1, the tyrosine residues at positions 734, 743 and 762 in the intracellular region are phosphorylated by the action of Src family kinase. The phosphorylated hCDCP1 provokes downstream signals as a result of phosphorylation of PKCδ, etc., and promotes anchorage-independent proliferation, degradation of extracellular matrix, cell migration, and epithelial-mesenchymal transition, thereby causing the metastasis of cancer cells. Moreover, it has been known that hCDCP1 interacts with various molecules such as EGFR and HER2, and promotes the proliferation and metastasis of cancer cells.

It has been reported that hCDCP1 is expressed in various cancer cells and normal tissues. For example, it has been reported that the hCDCP1 mRNA and protein are expressed in the cancer cells of prostate cancer, lung cancer, colorectal cancer, ovarian cancer and the like, in cell lines established from those cancer cells, and further, in the normal tissues of the colon, skin, small intestine, prostate and the like (see Patent Literature 1 and Patent Literature 2).

To date, several antibodies reacting against hCDCP1 have been known (hereinafter also referred to as "anti-human CDCP1 or anti-hCDCP1 antibodies").

Patent Literature 2 discloses an anti-hCDCP1 polyclonal antibody, and a screening method, a diagnostic method and a therapeutic method for ovarian cancer, in which the aforementioned antibody is used. Patent Literature 1 discloses an anti-hCDCP1 monoclonal antibody (clone name: 25A11). It is disclosed that an antibody formulated into ADC (antibody-drug conjugate), in which saporin is allowed to bind to 25A11, exhibits cytotoxicity against a PC3 cancer cell line *in vitro,* and that significant tumor growth inhibitory activity is exhibited by intravenous administration of this ADC antibody.

Moreover, Patent Literature 4 discloses a plurality of anti-hCDCP1 antibodies. It is disclosed that an anti-hCDCP1 antibody formulated into ADC with PBD (pyrrolobenzodiazepine) exhibits cytotoxicity against a prostate cancer cell line *in vitro,* and that an anti-hCDCP1 antibody formulated into ADC with MMAE (monomethyl auristatin E) exhibits antitumor activity against mouse xenograft models, into which a breast cancer cell line, a colorectal cancer cell line or a prostate cancer cell line has been transplanted.

Furthermore, Patent Literature 3 discloses four anti-hCDCP1 monoclonal antibodies (CUB1 antibody, CUB2 antibody, CUB3 antibody, and CUB4 antibody; all of which are derived from hybridoma clones CUB 1 to 4, respectively). It is disclosed that since these anti-hCDCP1 monoclonal antibodies bind to hCDCP1 proteins expressed in normal CD34-positive (CD34⁺) cells and normal CD133-positive (CD133⁺) cells, these antibodies can be used in separation and identification of hematopoietic stem cells, mesenchymal stem cells, neural stem cells, and other cells.

### Citation List

### Patent Literature

Patent Literature 1: International Publication WO2008/133851
Patent Literature 2: European Unexamined Patent Application Publication No. 1677875
Patent Literature 3: U.S. Unexamined Patent Application Publication No. 7,541,030
Patent Literature 4: International Publication WO2018/112334

### Non Patent Literature

Non Patent Literature 1: Mostageer et al., Oncogene 20: 4402-4408, 2001.
Non Patent Literature 2: Yang et al., Oncotarget 6: 43743-58, 2015.
Non Patent Literature 3: He et al., Oncoscience 3: 5-8, 2016.

### Summary of Invention

### Technical Problem

As mentioned above, it has been confirmed that the hCDCP1 mRNA and the hCDCP1 protein are expressed in normal tissues/cells and cancer tissues/cells. It has been disclosed that the CUB1 antibody disclosed in Patent Literature 3 strongly binds to normal CD34⁺/CD38⁻ bone marrow cells, as well as to hCDCP1-expressing K562 cells (human chronic myelogenous leukemia cells).

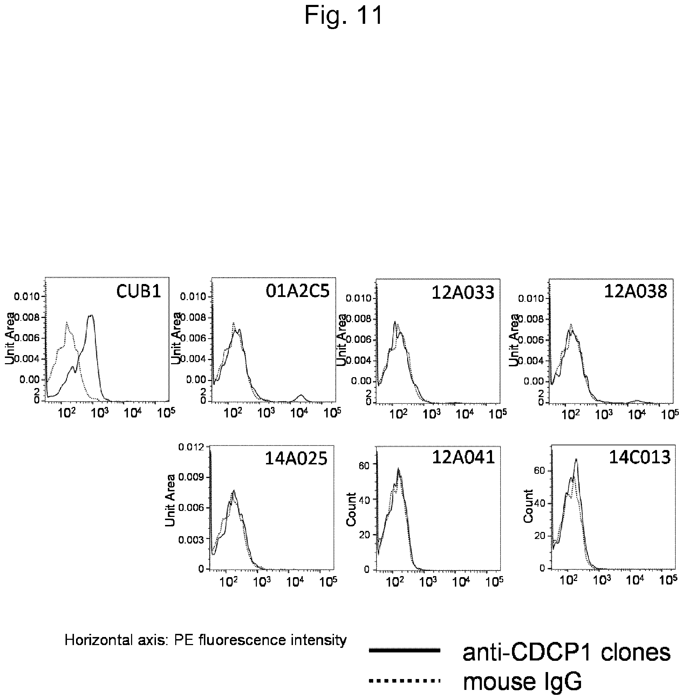

Further, the present inventors have confirmed that the 25A11-derived anti-hCDCP1 antibody disclosed in Patent Literature 2, the CUB4 antibody disclosed in Patent Literature 3, and the anti-hCDCP1 antibody commercially available from BioLegend (clone name: CUB1) strongly bind to normal CD34-positive bone marrow cells, as well as to various types of cancer cells that express hCDCP1 (see Figure 11, Figure 12, Figure 13A, Figure 15, and Figure 16).

Among bone marrow cells, a CD34-positive cell population comprises hematopoietic stem cells having ability to regenerate human blood cells as a whole, and CD34 is considered to be a cell surface marker of human hematopoietic stem cells.

The properties of these known antibodies to strongly bind to hematopoietic stem cells cause serious problems, when these anti-hCDCP1 antibodies are used as antitumor drugs. Specifically, it is highly likely that normal bone marrow cells, to which the anti-hCDCP1 antibody binds, are attacked by immune functions (e.g. ADCC activity), and that the functions of tissues, to which the cells belong, are inhibited and suppressed, so that serious side effects are generated. Furthermore, when the anti-hCDCP1 antibody is formulated into ADC, such an ADC antibody affects normal tissues and cells, so that more significant and serious adverse effects are given.

Therefore, it is an object of the present invention to provide an anti-hCDCP1 antibody that is unlikely to cause the above-described serious side effects.

### Solution to Problem

The present inventors have conducted studies regarding the antigen-binding properties of various anti-hCDCP1 antibodies. As a result, the present inventors have succeeded in preparing a novel anti-hCDCP1 antibody having properties, by which it binds to various cancer cells expressing hCDCP1, whereas it binds, at a relatively low level, to CD34-positive cells such as hematopoietic stem cells expressing hCDCP1.

Therefore, the present invention relates to an antibody that binds to CDCP1, which is characterized in that the antibody has low binding property to CD34-positive cells, and an antigen-binding fragment thereof.

### Advantageous Effects of Invention

According to the present invention, provided is an anti-hCDCP1 antibody having low binding property to CD34-positive cells (for example, CD34-positive bone marrow cells). The anti-hCDCP1 antibody according to the present invention exhibits antitumor activity, when it is formulated into ADC. Thus, the present anti-hCDCP1 antibody exhibits effects as an anticancer agent having few side effects.

### Brief Description of Drawings

[Figure 1] Figure 1 shows a histogram of gel permeation chromatography of a hCDCP1 extracellular domain purified protein.
[Figure 2] Figure 2 shows the results of SDS-PAGE performed on a hCDCP1 extracellular domain purified protein that has been cleaved with plasmin.
[Figure 3] Figure 3 shows the results of flow cytometric observation of the expression level of a hCDCP1 protein on the cell surface of Ba/F3 cells that forcibly express hCDCP1.
[Figure 4] Figure 4 shows the results obtained by cleaving by a trypsin treatment, CDCP1 on the cell surface of Ba/F3 cells that forcibly express hCDCP1. Figure 4A shows a change over time in the mean fluorescence intensity of PE in the cell population, which is measured by flow cytometry. Figure 4B shows the results of Western blotting performed on cleaved hCDCP1 molecules.
[Figure 5] Figure 5 shows the results of screening for anti-hCDCP1 antibody-producing hybridomas according to Cell-ELISA using PC3 cells and hCDCP1-deficient PC3 cells. The results of Cell-ELISA using culture supernatants of hybridomas produced from individual immune animals in Experiment 1, Experiment 2 and Experiment 3 (see the section of Examples) are shown in A, B, and C and D.
[Figure 6A] Figure 6 A shows CDR mutation introduction sites of sequences, from which mouse-human chimeric antibodies (mh12A041 series and mh14A025 series) have been produced.
[Figure 6B] Figure 6B shows CDR mutation introduction sites of sequences, from which mouse-human chimeric antibodies (mh14A043 series and mh14A063 series) have been produced.
[Figure 7] Figure 7 shows the results of flow cytometric observation of the reactivity of mouse-human chimeric antibodies to hCDCP1 forced expression cells.
[Figure 8] Figure 8 shows the results of flow cytometric observation of the reactivity of mouse-human chimeric antibodies to trypsin-treated hCDCP1 forced expression Ba/F3 cells.
[Figure 9] Figure 9 shows the results of flow cytometric observation of the reactivity of mouse-human chimeric antibodies to crab-eating macaque CDCP1.
[Figure 10A] Figure 10A shows the results of flow cytometric observation of the reactivity of mouse-human chimeric antibodies to the cancer cell lines (SK-MES-1, H358, MDA-MB-231, HCC1143, Capan-2, DLD-1, and OVCAR3).
[Figure 10B] Figure 10B shows the results of flow cytometric observation of the reactivity of mouse-human chimeric antibodies to the cancer cell lines (SK-OV-3, TFK-1, PC3, and DU145) and primary cultured cells derived from the normal tissues (HMEpC and NHEK).
[Figure 11] Figure 11 shows the results of flow cytometric observation, in which mouse antibodies produced by hybridomas are compared with one another, in terms of reactivity to healthy human bone marrow CD34-positive cells at a comparative antibody concentration of 10 µg/mL.
[Figure 12] Figure 12 shows the results of flow cytometric observation, in which mouse-human chimeric antibodies are compared with one another, in terms of reactivity to healthy human bone marrow CD34-positive cells at a comparative antibody concentration of 10 µg/mL.
[Figure 13] Figures 13A to C show the results of flow cytometric observation of the reactivity of biotinylated mouse-human chimeric antibodies to healthy human bone marrow CD34-positive cells. Figure 13D is a view showing a comparison made between the anti-RS virus antibody biotinylated antibody used in Figures 13A to C and a purified IgG biotinylated protein derived from normal human serum, in terms of reactivity to bone marrow CD34-positive cells at a comparative antibody concentration of 10 ng/mL.
[Figure 14] Figure 14 shows the results of flow cytometric observation of the reactivity of humanized anti-hCDCP1 antibodies to hCDCP1 forced expression Ba/F3 cells.
[Figure 15] Figure 15 shows the results of flow cytometric observation of the reactivity of biotinylated humanized anti-hCDCP1 antibodies and biotinylated comparative control antibodies to healthy human bone marrow CD34-positive cells at a comparative antibody concentration of 10 ng/mL.
[Figure 16] Figure 16 shows the results of flow cytometric observation of the concentration dependence of the reactivity of biotinylated humanized antibodies to healthy human bone marrow CD34-positive cells.
[Figure 17] Figure 17 shows the *in vitro* cytotoxicity of PBD-bound anti-hCDCP1 mouse-human chimeric antibody-drug conjugates against cancer cell lines and primary cultured cells derived from normal tissues.
[Figure 18] Figure 18 shows the *in vitro* cytotoxicity of PBD-bound humanized anti-hCDCP1 antibody-drug conjugates against cancer cell lines and normal human epidermal keratinocyte primary cultured cells.
[Figure 19] Figures 19A and B show the antitumor activity of PBD-bound anti-hCDCP1 mouse-human chimeric antibody-drug conjugates in PC3 cell line xenograft models (scid mouse models). Figures 19C and D show the antitumor activity of PBD-bound anti-hCDCP1 mouse-human chimeric antibody-drug conjugates in PC3 cell line xenograft models (nude mouse models).
[Figure 20] Figure 20A shows the antitumor activity of PBD-bound humanized anti-hCDCP1 antibody-drug conjugates in PC3 cell line xenograft models (nude mouse models). Figure 20B shows the antitumor activity of PBD-bound humanized anti-hCDCP1 antibody-drug conjugates in the colorectal cancer cell line HCT116 xenograft models.
[Figure 21] Figure 21 shows the antitumor activity of MMAE-bound humanized anti-hCDCP1 antibody-drug conjugates in the cell line HCT116 xenograft models.

### Description of Embodiments

A first embodiment of the present invention relates to an antibody that binds to human CDCP1, wherein the antibody is characterized in that its binding property to human CD34-positive (CD34⁺) cells is low (hereinafter also referred to as "the anti-hCDCP1 antibody of the present invention"), or an antigen-binding fragment thereof.

CD34-positive cells mean cells that express CD34 antigens on the cell surface thereof. CD34 is a single-chain transmembrane phosphorylated glycoprotein having a molecular weight of approximately 110 kDa, and CD34 has two domains having each different structures outside of a cell. Such CD34 is a surface antigen marker present on various somatic stem cells and is expressed in bone marrow-derived hematopoietic stem cells/endothelial progenitor cells, skeletal muscle satellite cells, hair follicle stem cells, adipose tissue mesenchymal stem cells, etc.

Herein, the CD34-positive cells may be, for example, hematopoietic stem cells that are able to differentiate into blood cells. Among such hematopoietic stem cells, CD34 is expressed at the highest level in the most undifferentiated hematopoietic stem cells, and as the hematopoietic stem cells differentiate into various cell strains, the expression level of CD34 decreases.

Herein, the "binding property" of the hCDCP1 antibody of the present invention to CD34-positive cells means the ability of the present anti-hCDCP1 antibody to bind to any site of CD34-positive cells (binding ability). In the first embodiment, the binding property of the anti-hCDCP1 antibody to human CD34-positive cells is relatively evaluated by comparing the binding ability of the anti-hCDCP1 antibody to human CD34-positive cells with the binding ability of non-specific human IgG to the CD34-positive cells. Herein, the "non-specific human IgG" means human-derived IgG that does not have specific reactivity to a human CDCP1 protein, and it specifically indicates a monoclonal antibody known to have no specific reactivity to human CDCP1, or more preferably, a mixture of a plurality of monoclonal antibodies having no specific reactivity to CDCP1, or more preferably, an IgG mixture purified and extracted from living human serum according to a means such as affinity chromatography. As a specific evaluation method, when the anti-hCDCP1 antibody shows the same level of binding ability as that of non-specific human IgG to human CD34-positive cells under conditions in which the anti-hCDCP1 antibody and the non-specific human IgG have an identical antibody concentration (hereinafter referred to as a "comparative antibody concentration") (i.e. under conditions in which the concentration of the anti-hCDCP1 antibody is identical to the concentration of the non-specific human IgG), the binding property of the anti-hCDCP1 antibody to human CD34-positive cells is evaluated to be low. For example, when the anti-hCDCP1 antibody does not show statistically significantly high binding ability to human CD34-positive cells, compared with the non-specific human IgG, the binding property of the anti-hCDCP1 antibody to human CD34-positive cells may be evaluated to be low. On the other hand, when the anti-hCDCP1 antibody shows high binding ability to human CD34-positive cells, compared with the non-specific human IgG, the binding property of the anti-hCDCP1 antibody to human CD34-positive cells is evaluated to be high. For example, when the anti-hCDCP1 antibody shows statistically significantly high binding ability to human CD34-positive cells, compared with the non-specific human IgG, the binding property of the anti-hCDCP1 antibody to human CD34-positive cells may be evaluated to be high.

In addition, from another viewpoint, an antibody having binding property to CD34-positive cells that is lower than or equivalent to the binding property of a certain antibody serving as an indicator is included in the scope of the invention of the present application. The aforementioned antibody may be, for example, an antibody having binding property to CD34-positive cells that is equivalent to or lower than an antibody (clone name: h12A041VH1A/VL) having binding property to CD34-positive cells that is lower than that of CUB4 as a known antibody.

Herein, the "comparative antibody concentration" is not particularly limited, but it means a specific concentration point, and it is, for example, any concentration of 10 ng/ml or more, any concentration of 100 ng/ml or more, any concentration of 1 µg/ml or more, and more preferably, any concentration of 10 µg/ml or more.

In addition, herein, the concentration point of 10 ng/ml is a concentration in which CUB4 sufficiently reacts in the present test system, namely, a concentration in which CUB4 sufficiently binds to CD34-positive cells. Thus, comparative evaluation of binding property can be sufficiently carried out in this concentration point.

Moreover, the binding property of the anti-hCDCP1 antibody or the non-specific human IgG to CD34-positive cells can be evaluated, for example, by flow cytometric analysis, an ELISA method, a RIA method, a surface plasmon resonance method, etc., although the evaluation method is not particularly limited thereto.

For example, the anti-hCDCP1 antibody of the present invention can be prepared as follows. From among antibodies produced by using, as antigens, the entire extracellular domain of human CDCP1 or a part thereof, or cells expressing the entire extracellular domain of human CDCP1 or a part thereof on the surface thereof, antibodies reacting to human CDCP1 expressed on cancer cells are screened, and from the screened antibody group, an antibody having low reactivity to CD34-positive cell fractions of healthy human bone marrow cells is selected, so that a desired antibody can be prepared.

The "antibody" used in the present description is not particularly limited in terms of a preparation method thereof and a structure thereof, and examples of the present antibody may include all "antibodies" that each bind to a desired antigen based on desired properties, such as, for example, a monoclonal antibody, a polyclonal antibody, or a nanoantibody.

When the anti-hCDCP1 antibody of the present invention is a polyclonal antibody, the anti-hCDCP1 antibody can be prepared, for example, by injecting a mixture of an antigen and an adjuvant into an immune animal (which, for example, includes, but is not limited to, a rabbit, a goat, sheep, a chicken, a Guinea pig, a mouse, a rat, a pig, etc.). In general, an antigen and/or an adjuvant are injected into the subcutis or abdominal cavity of such an immune animal several times. Examples of the adjuvant may include, but are not limited to, complete Freund and monophosphoryl lipid A - trehalose dicorynomycolate (MPL-TDM). After immunization with the antigen, the anti-hCDCP1 antibody can be purified from the serum derived from the immune animal by a conventional method (for example, a method using Sepharose that carries Protein A, etc.).

On the other hand, when the anti-hCDCP1 antibody of the present invention is a monoclonal antibody, the anti-hCDCP1 antibody can be produced, for example, as follows. Besides, the term "monoclonal" is used in the present description to suggest the properties of an antibody obtained from a population of substantially uniform antibodies (i.e. an antibody population, in which the amino acid sequences of heavy chains and light chains constituting the antibodies are identical to one another), and thus, it does not mean that the antibody is produced by a specific method (e.g. a hybridoma method, etc.).

Examples of the method of producing a monoclonal antibody may include a hybridoma method (Kohler and Milstein, Nature 256, 495, 1975) and a recombination method (U.S. Patent No. 4,816,567). Otherwise, the anti-hCDCP1 antibody of the present invention may be isolated from a phage antibody library (for example, Clackson et al., Nature 352, 624-628, 1991; Marks et al., J. Mol. Biol. 222, 581-597, 1991; etc.) or a cell library (for example, Japanese Patent No. 4214234; Seo et al., Nature Biotech., 23, 731-735, 2005; etc.). More specifically, when a monoclonal antibody is prepared by applying a hybridoma method, the preparation method includes, for example, the following 4 steps: (i) immunizing an immune animal with an antigen, (ii) recovering monoclonal antibody-secreting (or potentially secreting) lymphocytes, (iii) fusing the lymphocytes with immortalized cells, and (iv) selecting cells that secrete a desired monoclonal antibody. Examples of the immune animal that can be selected herein may include a mouse, a rat, a Guinea pig, a hamster, and a rabbit. After completion of the immunization, in order to establish hybridoma cells, lymphocytes obtained from a host animal are fused with an immortalized cell line, using a fusion agent such as polyethylene glycol, or an electrical fusion method. As fusion cells, for example, a rat or mouse myeloma cell line is used. After completion of the cell fusion, the cells are allowed to grow in a suitable medium containing one or more substrates that inhibit the growth or survival of unfused lymphocytes and immortalized cell line. According to an ordinary technique, parent cells that lack the enzyme, hypoxanthine-guanine phosphoribosyl transferase (HGPRT or HPRT), are used. In this case, hypoxanthine, aminopterin and thymidine are added to a medium that inhibits HGPRT-deficient cells and accepts the growth of hybridomas (i.e. HAT medium). From the thus obtained hybridomas, hybridomas generating desired antibodies are selected, and thereafter, a monoclonal antibody of interest can be obtained from a medium in which the selected hybridomas grow, according to an ordinary method.

The thus prepared hybridomas are cultured *in vitro,* or are cultured *in vivo* in the ascites fluid of a mouse, a rat, a Guinea pig, a hamster, etc., and an antibody of interest can be then prepared from the culture supernatant, or from the ascites fluid.

Nanoantibody is a polypeptide consisting of a variable region of an antibody heavy chain (i.e. a variable domain of the heavy chain of heavy chain antibody (VHH)). In general, an antibody of a human or the like is composed of heavy and light chains. However, animals of family Camelidae, such as llamas, alpacas and camels, produce single-chain antibodies (heavy chain antibodies) consisting only of heavy chains. Such a heavy chain antibody can recognize a target antigen and can bind thereto, as in the case of a common antibody consisting of heavy and light chains. The variable region of a heavy chain antibody is the smallest unit that has a binding affinity to an antigen, and this variable region fragment is called a "nanoantibody." Nanoantibodies have high heat resistance, digestion resistance, and room temperature stability, and can be easily prepared in large quantities by a genetic engineering technique.

A nanoantibody can be produced, for example, as follows. An animal of family Camelidae is immunized with an antigen, and the presence or absence of an antibody of interest is then detected from the collected serum. Thereafter, cDNA is prepared from RNA derived from the peripheral blood lymphocytes of an immune animal, in which a desired antibody titer is detected. A DNA fragment encoding VHH is amplified from the obtained cDNA, and the amplified DNA fragment is then inserted into a phagemid to prepare a VHH phagemid library. A desired nanoantibody can be prepared from the prepared VHH phagemid library through several screenings.

The anti-hCDCP1 antibody of the present invention may be a genetically engineered antibody. Such a genetically engineered antibody is not limited, and examples thereof may include a human antibody, and a chimeric antibody with a human antibody. The chimeric antibody is, for example, an antibody, in which a variable region derived from a different animal species is linked with a constant region derived from another different animal species (for example, an antibody, in which a variable region of a mouse-derived antibody is bound to a constant region derived from a human) (for example, Proc. Natl. Acad. Sci. U.S.A. 81, 6851-6855 (1984), etc.). The chimeric antibody can be easily constructed by genetic recombination technology.

The humanized antibody is an antibody that has a human-derived sequence in the framework region (FR) thereof and has a complementarity determining region (CDR) consisting of a sequence derived from another animal species (for example, a mouse, etc.). Humanized antibody is produced by transplanting CDRs from the variable regions of antibody derived from another animal species, for the first example, mouse, into human antibody variable regions, so that the heavy chain and light chain variable regions of the human antibody are reconstituted. Thereafter, the humanized reconstituted human antibody variable regions are ligated to humanized antibody constant regions, so that a humanized antibody can be produced. The method for producing such a humanized antibody is publicly known in the present technical field (e.g. Proc. Natl. Acad. Sci. USA, 86: 10029-10033 (1989), etc.).

The antigen-binding fragment of the present invention is a partial region of the antibody of the present invention, which is an antibody fragment that binds to human CDCP1. Examples of such an antigen-binding fragment may include Fab, Fab', F(ab')₂, Fv (a variable fragment of an antibody), a single chain antibody (a heavy chain, a light chain, a heavy chain variable region, a light chain variable region, a nanoantibody, etc.), scFv (single chain Fv), a diabody (an scFv dimer), dsFv (disulfide-stabilized Fv), and a peptide comprising the CDR of the antibody of the present invention, at least, as a part thereof.

Fab is an antibody fragment having antigen-binding activity, in which about a half of the N-terminal side of a heavy chain and a light chain as a whole are bound to each other via a disulfide bond, among fragments obtained by treating an antibody molecule with the proteolytic enzyme papain. Such Fab can be produced by treating an antibody molecule with papain to obtain a fragment, and also, for example, by constructing a suitable expression vector into which DNA encoding Fab is inserted, then introducing this vector into suitable host cells (e.g. mammalian cells such as CHO cells, yeast cells, insect cells, etc.), and then allowing Fab to express in the cells.

F(ab')2 is an antibody fragment having antigen-binding activity, which is obtained by treating an antibody molecule with the proteolytic enzyme pepsin, and which is slightly larger than a fragment in which Fab is bound via a disulfide bond in the hinge region. Such F(ab')₂ can be produced by treating an antibody molecule with pepsin to obtain a fragment, or via a thioether bond or a disulfide bond, or further, by a genetic engineering technique, as in the case of Fab.

Fab' is an antibody fragment having antigen-binding activity, in which the disulfide bond in the hinge region of the above-described F(ab')₂ is cleaved. Such Fab' can also be produced by a genetic engineering technique, as in the case of Fab etc..
scFv is a VH-linker-VL or VL-linker-VH polypeptide, in which one heavy chain variable region (VH) and one light chain variable region (VL) are linked to each other using a suitable peptide linker, and it is an antibody fragment having antigen-binding activity. Such ScFv can be produced by obtaining cDNAs encoding the heavy and light chain variable regions of an antibody, and then performing a genetic engineering technique.

Diabody is an antibody fragment having a divalent antigen-binding activity, in which scFv is dimerized. The divalent antigen-binding activity may be an identical antigen-binding activity, or one of them may be a different antigen-binding activity. Such a diabody can be produced by obtaining cDNAs encoding the heavy chain and light chain variable regions of an antibody, then constructing cDNA encoding scFv, in which the heavy chain variable region and the light chain variable region are linked to each other by a peptide linker, and then performing a genetic engineering technique.
dsFv refers to polypeptides, in which one amino acid residue in each of the heavy chain variable region and the light chain variable region is replaced with a cysteine residue, which are bound to each other via a disulfide bond between the cysteine residues. The amino acid residue to be replaced with the cysteine residue can be selected based on the prediction of the three-dimensional structure of the antibody. Such dsFv can be produced by obtaining cDNAs encoding the heavy chain and light chain variable regions of an antibody, then constructing DNA encoding the dsFv, and then performing a genetic engineering technique.

A peptide comprising a CDR is configured to comprise at least one region of the CDRs (CDR1 to 3) of a heavy or a light chain. A plurality of peptides each comprising a CDR can be bound to one another, directly or via a suitable peptide linker. Such a peptide comprising a CDR can be produced by constructing DNA encoding the CDR of the heavy chain or light chain of an antibody, and inserting the constructed DNA into an expression vector. Herein, the type of the vector is not particularly limited, and it may be appropriately selected, depending on the types of host cells into which the vector is to be introduced, etc. Thereafter, the peptide comprising a CDR can be produced by introducing the expression vector comprising the DNA into suitable host cells (e.g. mammalian cells such as CHO cells, yeast cells, insect cells, etc.) for allowing it to express as an antibody. Alternatively, the peptide comprising a CDR can also be produced by a chemical synthesis method such as an Fmoc method (fluorenylmethyloxycarbonyl method) and a tBoc method (t-butyloxycarbonyl method).

In general, in the case of a human antibody (a complete human antibody), a hypervariable region that is the antigen binding site of a V region, other parts of the V region, and a constant region have the same structures as those of the antibody of a human. Such a human antibody can be easily produced by a person skilled in the art according to a known technique. The human antibody can be obtained by, for example, a method using a human antibody-producing mouse having a human chromosome fragment containing the H chain and L chain genes of the human antibody (e.g. Tomizuka et al., Proc. Natl. Acad. Sci. USA, (2000) 97, 722-727, etc.) or a method of obtaining a human antibody derived from a phage display selected from a human antibody library (see, for example, Siriwardena et al., Opthalmology, (2002) 109 (3), 427-431, etc.).

A multispecific antibody can be constructed using the antigen-binding fragment of the present invention. Multispecificity means that an antibody has binding specificity to two or more antigens, and may be, for example, the form of a protein containing a monoclonal antibody having binding specificity to two or more antigens or an antigen-binding fragment thereof. Such multispecificity is achieved by a person skilled in the art according to a known technique. As methods of constructing multispecificity, there have been developed multiple methods, which are classified into a technique of constructing an asymmetric IgG, in which two different types of antibody heavy chain molecules are subjected to protein engineering operations so that they form a heterodimer, and a technique of ligating to each other, antigen-binding fragments, which are obtained from an antibody, or ligating such an antigen-binding fragment to another antibody molecule. As an example of a specific construction method, the following publication can be, for example, referred to: Kontermann, R. E., & Brinkmann, U. (2015). Bispecific antibodies. Drug Discovery Today, 20(7), 838-847.

Examples of the anti-hCDCP1 antibody of the present invention and the antigen-binding fragment thereof may include antibodies, which are characterized in that the amino acid sequences of CDRs (complementarity determining regions) 1 to 3 thereof satisfy any of the following (A) to (S), and antigen-binding fragments thereof.
(A) An antibody having:
   heavy chain CDR1 comprising the amino acid sequence as set forth in SEQ ID NO: 25,
   heavy chain CDR2 comprising the amino acid sequence as set forth in SEQ ID NO: 26,
   heavy chain CDR3 comprising the amino acid sequence as set forth in SEQ ID NO: 27,
   light chain CDR1 comprising the amino acid sequence as set forth in SEQ ID NO: 29,
   light chain CDR2 comprising the amino acid sequence as set forth in SEQ ID NO: 30, and
   light chain CDR3 comprising the amino acid sequence as set forth in SEQ ID NO: 31.
(B) An antibody having:
   heavy chain CDR1 comprising the amino acid sequence as set forth in SEQ ID NO: 33,
   heavy chain CDR2 comprising the amino acid sequence as set forth in SEQ ID NO: 34,
   heavy chain CDR3 comprising the amino acid sequence as set forth in SEQ ID NO: 35,
   light chain CDR1 comprising the amino acid sequence as set forth in SEQ ID NO: 37,
   light chain CDR2 comprising the amino acid sequence as set forth in SEQ ID NO: 38, and
   light chain CDR3 comprising the amino acid sequence as set forth in SEQ ID NO: 39.
(C) An antibody having:
   heavy chain CDR1 comprising the amino acid sequence as set forth in SEQ ID NO: 41,
   heavy chain CDR2 comprising the amino acid sequence as set forth in SEQ ID NO: 42,
   heavy chain CDR3 comprising the amino acid sequence as set forth in SEQ ID NO: 43,
   light chain CDR1 comprising the amino acid sequence as set forth in SEQ ID NO: 45,
   light chain CDR2 comprising the amino acid sequence as set forth in SEQ ID NO: 46, and
   light chain CDR3 comprising the amino acid sequence as set forth in SEQ ID NO: 47.
(D) An antibody having:
   heavy chain CDR1 comprising the amino acid sequence as set forth in SEQ ID NO: 49,
   heavy chain CDR2 comprising the amino acid sequence as set forth in SEQ ID NO: 50,
   heavy chain CDR3 comprising the amino acid sequence as set forth in SEQ ID NO: 51,
   light chain CDR1 comprising the amino acid sequence as set forth in SEQ ID NO: 53,
   light chain CDR2 comprising the amino acid sequence as set forth in SEQ ID NO: 54, and
   light chain CDR3 comprising the amino acid sequence as set forth in SEQ ID NO: 55.
(E) An antibody having:
   heavy chain CDR1 comprising the amino acid sequence as set forth in SEQ ID NO: 57,
   heavy chain CDR2 comprising the amino acid sequence as set forth in SEQ ID NO: 58,
   heavy chain CDR3 comprising the amino acid sequence as set forth in SEQ ID NO: 59,
   light chain CDR1 comprising the amino acid sequence as set forth in SEQ ID NO: 61,
   light chain CDR2 comprising the amino acid sequence as set forth in SEQ ID NO: 62, and
   light chain CDR3 comprising the amino acid sequence as set forth in SEQ ID NO: 63.
(F) An antibody having:
   heavy chain CDR1 comprising the amino acid sequence as set forth in SEQ ID NO: 65,
   heavy chain CDR2 comprising the amino acid sequence as set forth in SEQ ID NO: 66,
   heavy chain CDR3 comprising the amino acid sequence as set forth in SEQ ID NO: 67,
   light chain CDR1 comprising the amino acid sequence as set forth in SEQ ID NO: 69,
   light chain CDR2 comprising the amino acid sequence as set forth in SEQ ID NO: 70, and
   light chain CDR3 comprising the amino acid sequence as set forth in SEQ ID NO: 71.
(G) An antibody having:
   heavy chain CDR1 comprising the amino acid sequence as set forth in SEQ ID NO: 73,
   heavy chain CDR2 comprising the amino acid sequence as set forth in SEQ ID NO: 74,
   heavy chain CDR3 comprising the amino acid sequence as set forth in SEQ ID NO: 75,
   light chain CDR1 comprising the amino acid sequence as set forth in SEQ ID NO: 77,
   light chain CDR2 comprising the amino acid sequence as set forth in SEQ ID NO: 78, and
   light chain CDR3 comprising the amino acid sequence as set forth in SEQ ID NO: 79.
(H) An antibody having:
   heavy chain CDR1 comprising the amino acid sequence as set forth in SEQ ID NO: 81,
   heavy chain CDR2 comprising the amino acid sequence as set forth in SEQ ID NO: 82,
   heavy chain CDR3 comprising the amino acid sequence as set forth in SEQ ID NO: 83,
   light chain CDR1 comprising the amino acid sequence as set forth in SEQ ID NO: 85,
   light chain CDR2 comprising the amino acid sequence as set forth in SEQ ID NO: 86, and
   light chain CDR3 comprising the amino acid sequence as set forth in SEQ ID NO: 87.
(I) An antibody having:
   heavy chain CDR1 comprising the amino acid sequence as set forth in SEQ ID NO: 89,
   heavy chain CDR2 comprising the amino acid sequence as set forth in SEQ ID NO: 90,
   heavy chain CDR3 comprising the amino acid sequence as set forth in SEQ ID NO: 91,
   light chain CDR1 comprising the amino acid sequence as set forth in SEQ ID NO: 93,
   light chain CDR2 comprising the amino acid sequence as set forth in SEQ ID NO: 94, and
   light chain CDR3 comprising the amino acid sequence as set forth in SEQ ID NO: 95.
(J) An antibody having:
   heavy chain CDR1 comprising the amino acid sequence as set forth in SEQ ID NO: 97,
   heavy chain CDR2 comprising the amino acid sequence as set forth in SEQ ID NO: 98,
   heavy chain CDR3 comprising the amino acid sequence as set forth in SEQ ID NO: 99,
   light chain CDR1 comprising the amino acid sequence as set forth in SEQ ID NO: 101,
   light chain CDR2 comprising the amino acid sequence as set forth in SEQ ID NO: 102, and
   light chain CDR3 comprising the amino acid sequence as set forth in SEQ ID NO: 103.
(K) An antibody having:
   heavy chain CDR1 comprising the amino acid sequence as set forth in SEQ ID NO: 105,
   heavy chain CDR2 comprising the amino acid sequence as set forth in SEQ ID NO: 106,
   heavy chain CDR3 comprising the amino acid sequence as set forth in SEQ ID NO: 107,
   light chain CDR1 comprising the amino acid sequence as set forth in SEQ ID NO: 109, light chain CDR2 comprising the amino acid sequence as set forth in SEQ ID NO: 110, and
   light chain CDR3 comprising the amino acid sequence as set forth in SEQ ID NO: 111.
(L) An antibody having:
   heavy chain CDR1 comprising the amino acid sequence as set forth in SEQ ID NO: 49,
   heavy chain CDR2 comprising the amino acid sequence as set forth in SEQ ID NO: 50,
   heavy chain CDR3 comprising the amino acid sequence as set forth in SEQ ID NO: 51,
   light chain CDR1 comprising the amino acid sequence as set forth in SEQ ID NO: 53,
   light chain CDR2 comprising the amino acid sequence as set forth in SEQ ID NO: 54, and
   light chain CDR3 comprising the amino acid sequence as set forth in SEQ ID NO: 55.
(M) The antibody satisfying the above (L), which has heavy chain and light chain variable regions, in which the amino acid at positions 28 and 29 of the light chain and the amino acid at position 102 of the heavy chain according to Kabat definition are substituted with other amino acids. Herein, other amino acids are not limited, but examples of such other amino acids substituted for the amino acid at position 28 of the light chain may include serine, threonine, alanine, glycine, leucine, isoleucine, valine, phenylalanine, arginine, tryptophan, histidine, methionine, glutamine, glutamic acid, lysine, and tyrosine. It is preferably serine. Examples of such other amino acids substituted for the amino acid at position 29 of the light chain may include alanine, aspartic acid, glutamic acid, methionine, arginine, leucine, isoleucine, valine, and lysine. It is preferably alanine. Examples of such other amino acids substituted for the amino acid at position 102 of the heavy chain may include alanine, glutamic acid, serine, threonine, glycine, leucine, isoleucine, valine, phenylalanine, tryptophan, methionine, lysine, histidine, and glutamine. It is preferably serine.
(N) An antibody having:
   heavy chain CDR1 comprising the amino acid sequence as set forth in SEQ ID NO: 65,
   heavy chain CDR2 comprising the amino acid sequence as set forth in SEQ ID NO: 66,
   heavy chain CDR3 comprising the amino acid sequence as set forth in SEQ ID NO: 67,
   light chain CDR1 comprising the amino acid sequence as set forth in SEQ ID NO: 69,
   light chain CDR2 comprising the amino acid sequence as set forth in SEQ ID NO: 70, and
   light chain CDR3 comprising the amino acid sequence as set forth in SEQ ID NO: 71.
(O) The antibody satisfying the above (N), which has a heavy chain variable region, in which the amino acids at position 54 and 55 of the heavy chain according to Kabat definition are substituted with other amino acids. Herein, other amino acids are not limited, but examples of such other amino acids substituted for the amino acid at position 54 of the heavy chain may include serine, threonine, alanine, glycine, leucine, isoleucine, valine, phenylalanine, arginine, tryptophan, histidine, methionine, glutamine, glutamic acid, lysine, and tyrosine. Preferred examples thereof may include serine, phenylalanine, arginine, threonine, and tryptophan. Examples of such other amino acids substituted for the amino acid at position 55 of the heavy chain may include alanine, aspartic acid, glutamic acid, methionine, arginine, leucine, isoleucine, valine, and lysine. Preferred examples thereof may include alanine, aspartic acid, glutamic acid, leucine, and methionine.
(P) An antibody having:
   heavy chain CDR1 comprising the amino acid sequence as set forth in SEQ ID NO: 73,
   heavy chain CDR2 comprising the amino acid sequence as set forth in SEQ ID NO: 74,
   heavy chain CDR3 comprising the amino acid sequence as set forth in SEQ ID NO: 75,
   light chain CDR1 comprising the amino acid sequence as set forth in SEQ ID NO: 77,
   light chain CDR2 comprising the amino acid sequence as set forth in SEQ ID NO: 78, and
   light chain CDR3 comprising the amino acid sequence as set forth in SEQ ID NO: 79.
(Q) The antibody satisfying the above (P), which has heavy chain and light chain variable regions, in which the amino acids at position 54 and 55 of the heavy chain and the amino acid at position 33 of the light chain according to Kabat definition are substituted with other amino acids. Herein, other amino acids are not limited, but examples of such other amino acids substituted for the amino acid at position 54 of the heavy chain may include serine, threonine, alanine, glycine, leucine, isoleucine, valine, phenylalanine, arginine, tryptophan, histidine, methionine, glutamine, glutamic acid, lysine, and tyrosine. It is preferably serine. Examples of such other amino acids substituted for the amino acid at position 55 of the heavy chain may include alanine, aspartic acid, glutamic acid, methionine, arginine, leucine, isoleucine, valine, and lysine. It is preferably alanine. Examples of such other amino acids substituted for the amino acid at position 33 of the light chain may include serine, threonine, alanine, glycine, leucine, isoleucine, valine, phenylalanine, arginine, tryptophan, asparagine, aspartic acid, glutamine, glutamic acid, tyrosine, lysine, and histidine. It is preferably leucine.
(R) An antibody having:
   heavy chain CDR1 comprising the amino acid sequence as set forth in SEQ ID NO: 89,
   heavy chain CDR2 comprising the amino acid sequence as set forth in SEQ ID NO: 90,
   heavy chain CDR3 comprising the amino acid sequence as set forth in SEQ ID NO: 91,
   light chain CDR1 comprising the amino acid sequence as set forth in SEQ ID NO: 93,
   light chain CDR2 comprising the amino acid sequence as set forth in SEQ ID NO: 94, and
   light chain CDR3 comprising the amino acid sequence as set forth in SEQ ID NO: 95.
(S) The antibody satisfying the above (R), which has heavy chain and light chain variable regions, in which the amino acids at positions 52a and 53 of the heavy chain and the amino acid at position 33 of the light chain according to Kabat definition are substituted with other amino acids. Herein, other amino acids are not limited, but examples of such other amino acids substituted for the amino acid at position 52a of the heavy chain may include serine, threonine, alanine, glycine, leucine, isoleucine, valine, phenylalanine, arginine, tryptophan, histidine, methionine, glutamine, glutamic acid, lysine, and tyrosine. It is preferably serine. Examples of such other amino acids substituted for the amino acid at position 53 of the heavy chain may include alanine, aspartic acid, glutamic acid, methionine, arginine, leucine, isoleucine, valine, and lysine. It is preferably alanine. Examples of such other amino acids substituted for the amino acid at position 33 of the light chain may include serine, threonine, alanine, glycine, leucine, isoleucine, valine, phenylalanine, arginine, tryptophan, asparagine, aspartic acid, glutamine, glutamic acid, tyrosine, lysine, and histidine. It is preferably leucine.

Furthermore, examples of the anti-hCDCP1 antibody of the present invention and the antigen-binding fragment thereof may further include antibodies, which are characterized in that the amino acid sequences of CDRs 1 to 3 of the heavy chain variable region thereof satisfy either following (T) or (U), and the amino acid sequences of CDRs 1 to 3 of the light chain variable region thereof satisfy any of the following (t) to (v), and antigen-binding fragments thereof.
(T) An antibody having:
   heavy chain CDR1 comprising the amino acid sequence as set forth in SEQ ID NO: 49,
   heavy chain CDR2 comprising the amino acid sequence as set forth in SEQ ID NO: 50, and
   heavy chain CDR3 comprising the amino acid sequence as set forth in SEQ ID NO: 113; or
(U) an antibody having:
   heavy chain CDR1 comprising the amino acid sequence as set forth in SEQ ID NO: 183,
   heavy chain CDR2 comprising the amino acid sequence as set forth in SEQ ID NO: 50, and
   heavy chain CDR3 comprising the amino acid sequence as set forth in SEQ ID NO: 113.
(t) An antibody having:
   light chain CDR1 comprising the amino acid sequence as set forth in SEQ ID NO: 117,
   light chain CDR2 comprising the amino acid sequence as set forth in SEQ ID NO: 54, and
   light chain CDR3 comprising the amino acid sequence as set forth in SEQ ID NO: 55;
(u) an antibody having:
   light chain CDR1 comprising the amino acid sequence as set forth in SEQ ID NO: 186,
   light chain CDR2 comprising the amino acid sequence as set forth in SEQ ID NO: 54, and
   light chain CDR3 comprising the amino acid sequence as set forth in SEQ ID NO: 55; and
(v) an antibody having:
   light chain CDR1 comprising the amino acid sequence as set forth in SEQ ID NO: 188,
   light chain CDR2 comprising the amino acid sequence as set forth in SEQ ID NO: 54, and
   light chain CDR3 comprising the amino acid sequence as set forth in SEQ ID NO: 55.

Still further, examples of the anti-hCDCP1 antibody of the present invention and the antigen-binding fragment thereof may further include: an antibody having any heavy chain variable region comprising the amino acid sequence as set forth in SEQ ID NO: 24, SEQ ID NO: 32, SEQ ID NO: 40, SEQ ID NO: 48, SEQ ID NO: 56, SEQ ID NO: 64, SEQ ID NO: 72, SEQ ID NO: 80, SEQ ID NO: 88, SEQ ID NO: 96, SEQ ID NO: 104, SEQ ID NO: 112, SEQ ID NO: 114, SEQ ID NO: 124, SEQ ID NO: 126, SEQ ID NO: 128, SEQ ID NO: 130, SEQ ID NO: 132, SEQ ID NO: 134, SEQ ID NO: 136, SEQ ID NO: 138, SEQ ID NO: 140, SEQ ID NO: 142, SEQ ID NO: 144, SEQ ID NO: 146, SEQ ID NO: 150, SEQ ID NO: 152, SEQ ID NO: 169, SEQ ID NO: 170, SEQ ID NO: 172, SEQ ID NO: 173, SEQ ID NO: 177, SEQ ID NO: 178, SEQ ID NO: 179, SEQ ID NO: 182 or SEQ ID NO: 184, and any light chain variable region comprising the amino acid sequence as set forth in SEQ ID NO: 28, SEQ ID NO: 36, SEQ ID NO: 44, SEQ ID NO: 52, SEQ ID NO: 60, SEQ ID NO: 68, SEQ ID NO: 76, SEQ ID NO: 84, SEQ ID NO: 92, SEQ ID NO: 100, SEQ ID NO: 108, SEQ ID NO: 116, SEQ ID NO: 118, SEQ ID NO: 120, SEQ ID NO: 122, SEQ ID NO: 148, SEQ ID NO: 154, SEQ ID NO: 155, SEQ ID NO: 171, SEQ ID NO: 174, SEQ ID NO: 175, SEQ ID NO: 176, SEQ ID NO: 180, SEQ ID NO: 181, SEQ ID NO: 185 or SEQ ID NO: 187; an antibody consisting of an amino acid sequence having an amino acid sequence identity of about 70% or more, preferably about 80% or more, about 81% or more, about 82% or more, about 83% or more, about 84% or more, about 85% or more, about 86% or more, about 87% or more, about 88% or more, about 89% or more, more preferably about 90% or more, about 91% or more, about 92% or more, about 93% or more, about 94% or more, about 95% or more, about 96% or more, about 97% or more, about 98% or more, and most preferably about 99% or more, to the amino acid sequence of each of a heavy chain variable region and/or a light chain variable region that constitute the aforementioned antibody, wherein the antibody is characterized in that it binds to human CDCP1 and its binding property to human CD34-positive cells is low; and the antigen-binding fragments of those antibodies.

A second embodiment of the present invention relates to: an antibody (hereinafter also referred to as "the competitive antibody of the present invention") that competitively inhibits the binding of the antibody according to the first embodiment (i.e. "the anti-hCDCP1 antibody of the present invention"), which is characterized in that it binds to human CDCP1 and its binding property to human CD34-positive cells is low, to human CDCP1; or an antigen-binding fragment thereof. The competitive antibody of the present invention can be prepared and obtained according to a competitive experiment publicly known to a person skilled in the art, etc. Specifically, when the binding of a first anti-hCDCP1 antibody (the antibody according to the embodiment) to human CDCP1 is competitively inhibited by a second anti-hCDCP1 antibody, it is determined that the first anti-hCDCP1 antibody and the second anti-hCDCP1 antibody bind to a substantially identical antigen site, or to antigen sites located extremely close to each other. When the binding property of the second anti-hCDCP1 antibody to CD34-positive cells is low, the second anti-hCDCP1 antibody is the competitive antibody of the present invention. As a method applied to such a competitive experiment, for example, a method using a Fab fragment, etc. is generally carried out in the present technical field. Please refer to, for example, WO95/11317, WO94/07922, WO2003/064473, WO2008/118356, WO2004/046733, and the like.

A third embodiment of the present invention relates to the antibody according to the first embodiment or the antibody according to the second embodiment, to which a substance having antitumor activity binds, or an antigen-binding fragment thereof.

A substance having antitumor activity, such as a drug, is allowed to bind to an antibody, so that a targeted therapy for cancer can be carried out (hereinafter, such a conjugate is referred to as "the conjugate of an antibody and a drug or the like of the present invention"). In this case, examples of the substance having antitumor activity may include, but are not limited to, cytotoxic drugs such as anticancer agents, radioisotopes, and substances that manipulate the immune system to indirectly induce antitumor activity.

In the third embodiment, a drug showing antitumor activity can be used, and such a conjugate is referred to as an "antibody-drug conjugate." Known examples of the drug showing antitumor activity used herein may include: tubulin inhibitors and microtubule polymerization inhibitors, such as Auristatins (MMAE, MMAF, etc.), Maytansines (DM1, DM4, etc.), Tubulysins, cryptophycins, and rhizoxin; antibiotics such as Calicheamicins, Doxorubicin, and anthracyclines; DNA synthesis inhibitors such as Duocarmycins, PBDs (Benzodiazepines), and IGNs (indolinobenzodiazepines); topoisomerase I inhibitors such as Canptothecin analogs (SN-38, DXd, etc.); RNA polymerase II inhibitors such as Amanitins; and RNA spliceosome inhibitors, such as spliceostatins and thailanstatins, but the examples are not limited thereto.

Moreover, as such a drug showing antitumor activity, a compound that is excited by light energy and expresses toxicity can also be used. Such an antibody-drug conjugate can be used in a therapeutic method called photoimmunotherapy (PIT), in which the antibody-drug conjugate is administered into a body and is allowed to bind to tumor cells, and then, light energy such as near infrared ray is given from outside of the body, so as to kill the tumor cells. The anti-hCDCP1 antibody of the present invention may also be used as an antibody in such photoimmunotherapy. IR700 or the like has been known as a compound used, but the compound used is not limited thereto.

Furthermore, there has been known radioimmunotherapy (RIT), in which a radioisotope is allowed to bind to an antibody, and cancer cells are then killed by using radiation emitted by the radioisotope. The anti-hCDCP1 antibody of the present invention can be used as an antibody in such radioimmunotherapy. Known examples of the radioisotopes used in the third embodiment of the present invention may include β ray nuclides such as ¹³¹I and ⁹⁰Y, and α ray nuclides such as ²¹³Bi, ²¹¹At, ²²⁵Ac, ²²³Ra, and ²¹²Pb, but the examples are not limited thereto.

Further, in the third embodiment of the present invention, there can be used a substance that directly or indirectly exhibits antitumor activity by manipulating the physiological action of a tumor itself, or tissues other than the tumor, such as an immune system. Taking the immune system as an example, examples of elements of the immune system to be manipulated may include: lymphoid cells, such as T cells, B cells, and NK cells; myeloid cells, such as monocytes, macrophages, dendritic cells, and granulocytes; and cells other than immune cells, which secrete and/or present a substance that gives an influence on these immune cells. Examples of the substance that manipulates these cells may include, but are not limited to, cancer vaccine peptides, cytokines (interleukins, interferons, colony stimulating factors (CSFs), etc.), hormones, and growth factors (TGF family, FGF family, IGF family, thrombopoietin, erythropoietin, etc.).

So far, a large number of chemical modification methods of allowing an antitumor substance to bind to an antibody have been known, and for example, the following methods have been known. Examples of the chemical modification methods may include: chemical modification methods involving a covalent bond to a lysine residue side chain, a covalent bond to a cysteine residue side chain, etc.; a method comprising introducing a non-natural amino acid into an antibody peptide chain, and then performing a site-specific chemical modification on the side chain; a method of performing a modification by using a specific amino acid sequence in an antibody or an enzyme reaction specific to a sugar chain to be modified; and a modification method using an enzyme catalyzing a peptide bond. In addition, in order to bind a drug or the like to a protein, it is general that a chemical modification is performed on the drug or the like, and that the drug or the like is used as a linker for protein binding. Many types of such chemical likers have been known, and the pharmacological action of a conjugate of an antibody or a drug or the like largely varies depending on the properties of such a chemical linker. For example, a hydrazone linker, a valine-citrulline linker, an SS bond linker, a pyrophosphoric acid linker or the like can be used to prepare an antibody-drug conjugate, in which the conjugate is cleaved by an enzyme or the like existing in a body, and the drug is separated from the antibody, thereby exhibiting high antitumor effects. On the other hand, as such a chemical linker, a chemical structure that cannot be cleaved *in vivo* can be generally used. A summary regarding the aforementioned methods is described in, for example, the following publication: Tsuchikama and An, (2018). Antibody-drug conjugates: recent advances in conjugation and linker chemistries. Protein and Cell, 9(1), 33-46.

The drug-antibody binding ratio (Drug Antibody Ratio: DAR) is a numerical value showing the number of drug molecules binding to a single antibody molecule in an antibody-drug conjugate. DAR is changed depending on a method of chemically binding a drug to an antibody, and the value of DAR is generally from 1 to 8. However, it is possible to produce an antibody-drug conjugate having DAR of 9 or greater, depending on the chemical binding manner.

A fourth embodiment of the present invention relates to a pharmaceutical composition for preventing or treating cancer (hereinafter also referred to as "the pharmaceutical composition of the present invention"), comprising the conjugate of an antibody and a drug or the like of the present invention according to the third embodiment, or an antigen-binding fragment thereof.

The pharmaceutical composition of the present invention may be administered in the form of a pharmaceutical composition, which comprises one or two or more pharmaceutical additives, as well as the conjugate of an antibody and a drug or the like of the present invention or an antigen-binding fragment thereof, used as an active ingredient. Moreover, the pharmaceutical composition according to the present embodiment may further comprise known other drugs.

It has been known that, when a therapeutic antibody that can be administered to humans is produced using the conjugate of an antibody and a drug or the like of the present invention, or an antigen-binding fragment thereof, a chemical modification frequently occurs after purification of the antibody in the production process thereof. In the present technical field, an action against such a chemical modification is recognized to be important for ensuring the quality of the purified antibody and safety. Specifically, it is predicted that the quality of the purified antibody will be deteriorated, when an amino acid sequence motif, which easily receives modifications such as N-type sugar chain modification, protein cleavage, deamidation, racemization and oxidation, is present in the antibody sequence comprising CDR regions. As such an amino acid sequence motif easily receiving modifications, for example, a motif comprising an asparagine residue and an aspartic acid residue has been known (see, for example, Sydow et al., (2014). Structure-based prediction of asparagine and aspartate degradation sites in antibody variable regions. PLoS ONE, 9(6), etc.). If such an amino acid motif can be modified while maintaining the activity of the antibody, the usefulness of the antibody can be further enhanced.

The pharmaceutical composition of the present invention may have a dosage form for either oral or parenteral administration, and the dosage form of the present pharmaceutical composition is not particularly limited. Examples of the dosage form may include tablets, capsules, granules, powder agents, syrups, suspensions, suppositories, ointments, creams, gelling agents, patches, inhalants, and injections. These preparations are produced according to ordinary methods. Liquid preparations may be dissolved or suspended in water or another suitable solvent at the time of use. In addition, tablets and granules may be coated by a publicly known method. Injections are prepared by dissolving the antibody of the present invention or a functional fragment thereof in water. As necessary, the antibody of the present invention or a functional fragment thereof may be dissolved in a normal saline or a glucose solution, and further, a buffer agent or a preservative may be added to such a solution.

The types of pharmaceutical additives used in production of the pharmaceutical composition of the present invention, the ratio of the pharmaceutical additives to the active ingredient, or a method for producing the pharmaceutical composition can be appropriately selected by a person skilled in the art, depending on the forms thereof. As such pharmaceutical additives, inorganic or organic substances, or solid or liquid substances can be used. In general, such pharmaceutical additives can be mixed in an amount, for example, from 0.1% by weight to 99.9% by weight, 1% by weight to 95% by weight, or 1% by weight to 90.0% by weight, based on the weight of the active ingredient. Specific examples of the pharmaceutical additives may include lactose, glucose, mannit, dextrin, cyclodextrin, starch, sucrose, magnesium aluminometasilicate, synthetic aluminum silicate, sodium carboxymethyl cellulose, hydroxypropyl starch, calcium carboxymethyl cellulose, ion exchange resin, methyl cellulose, gelatin, gum Arabic, hydroxypropyl cellulose, hydroxypropyl methyl cellulose, polyvinyl pyrrolidone, polyvinyl alcohol, light anhydrous silicic acid, magnesium stearate, talc, tragacanth, bentonite, veegum, titanium oxide, sorbitan fatty acid ester, sodium lauryl sulfate, glycerin, fatty acid glycerin ester, purified lanolin, glycerogelatin, polysorbate, macrogol, vegetable oil, wax, liquid paraffin, white petrolatum, fluorocarbon, nonionic surfactant, propylene glycol, and water.

In order to produce a solid preparation for use in oral administration, an active ingredient is mixed with excipient components, such as, for example, lactose, starch, crystalline cellulose, calcium lactate, or anhydrous silicic acid, to form a powder agent. Otherwise, as necessary, a binder such as white sugar, hydroxypropyl cellulose or polyvinyl pyrrolidone, a disintegrator such as carboxymethyl cellulose or calcium carboxymethyl cellulose, and the like are further added thereto, and the obtained mixture is then subj ected to wet or dry granulation to form a granule. In order to produce a tablet, such a powder agent or a granule is directly used, or a lubricant such as magnesium stearate or talc is added thereto, and they are then subj ected to tableting. Such a granules or a tablet can be coated with an enteric coating base material such as hydroxypropylmethyl cellulose phthalate or a methacrylic acid-methyl methacrylate polymer to form an enteric coated preparation. Otherwise, such a granule or tablet can be coated with ethyl cellulose, carnauba wax, or hydrogenated oil to form a prolonged action preparation. Moreover, in order to produce a capsule, a powder agent or a granule is filled into a hard capsule. Otherwise, an active ingredient is directly used, or is dissolved in glycerin, polyethylene glycol, sesame oil, olive oil or the like, and the obtained mixture is then coated with gelatin, so that a soft capsule can be prepared.

In order to produce an injection, an active ingredient is dissolved in distilled water for injection, together with, as necessary, a pH adjuster such as hydrochloric acid, sodium hydroxide, lactose, lactic acid, sodium, sodium monohydrogen phosphate or sodium dihydrogen phosphate, and a tonicity agent such as sodium chloride or glucose, and thereafter, the obtained solution is subjected to aseptic filtration, and is then filled into an ampoule. Otherwise, mannitol, dextrin, cyclodextrin, gelatin or the like is further added to the obtained solution, and the thus mixed solution is then subjected to vacuum - freeze drying, so that the injection may be prepared as an injection that is dissolved at the time of use. Alternatively, lecithin, polysorbate 80, polyoxyethylene hydrogenated castor oil, etc. can be added to the active ingredient, and they can be then emulsified in water to prepare an emulsion for injection.

In order to produce an agent for rectal administration, an active ingredient may be humidified and dissolved, together with a suppository base material such as cacao butter, fatty acid tri-, di- and mono-glyceride, or polyethylene glycol, and thereafter, the obtained mixture may be poured into a mold and may be then cooled. Otherwise, an active ingredient may be dissolved in polyethylene glycol, soybean oil or the like, and the obtained mixture may be then coated with a gelatin film or the like.

The applied dose and the number of doses of the pharmaceutical composition of the present invention are not particularly limited, and the applied dose and the number of doses can be selected, as appropriate, by a doctor's or a pharmacist's judgement, depending on conditions such as the purpose of prevention and/or treatment of deterioration/progression of a therapeutic target disease, the type of the disease, and the body weight, age, etc. of a patient.

In general, the daily dose of the pharmaceutical composition of the present invention for an adult by oral administration is approximately 0.01 to 1,000 mg (the weight of the active ingredient), and the pharmaceutical composition of the present invention can be administered once per day, or divided over several administrations per day. When the pharmaceutical composition of the present invention is used in the form of an injection, it is desired to continuously or intermittently administer the therapeutic agent or the like to an adult at a daily dose of 0.001 to 100 mg (the weight of the active ingredient).

Another form of the pharmaceutical composition of the present invention may be cytotoxic cells that express the antibody of the present invention or an antigen-binding fragment thereof on the cell surface thereof, such as T cells. Chimeric antigen receptor T-cell (CAR-T) therapy is a treatment method, in which T cells are allowed to express a fusion gene (chimeric antigen receptor gene) of an antigen-binding site of an antibody with a part of a T cell receptor, and the T cells are then introduced into the body of a cancer patient, so that the introduced T cells specifically attack the cancer cells, thereby providing antitumor activity to the cancer cells. A gene encoding the antibody of the present invention or an antigen-binding fragment thereof is used as a constitutional element of the above-described chimeric antigen receptor gene to construct the expressing T cells, so that a CAR-T therapy for specifically attacking a tumor expressing human CDCP1 molecules can be constructed.

The pharmaceutical composition of the present invention can attack and kill cancer cells, as long as the cancer cells express hCDCP1 on the cell surface thereof. Accordingly, the cancer as a therapeutic target of the pharmaceutical composition of the present invention may be any cancer, and is not particularly limited. Representative examples of the cancer may include: malignant tumors, such as hepatocellular carcinoma, bile duct cell carcinoma, renal cell carcinoma, squamous cell carcinoma, basal cell carcinoma, transitional cell carcinoma, adenocarcinoma, malignant gastrinoma, malignant melanoma, fibrosarcoma, myxosarcoma, liposarcoma, leiomyosarcoma, rhabdomyosarcoma, malignant teratoma, angiosarcoma, Kaposi's sarcoma, osteosarcoma, chondrosarcoma, lymphangiosarcoma, malignant meningioma, non-Hodgkin lymphoma, Hodgkin lymphoma, leukemia, and brain tumor; malignant neoplasms, such as epithelial cell-derived neoplasm (epithelial carcinoma), basal cell carcinomas, adenocarcinomas, lip cancer, oral cancer, esophageal cancer, gastrointestinal cancer such as small bowel cancer and stomach cancer, colon cancer, rectal cancer, bladder cancer, pancreatic cancer, ovarian cancer, cervical cancer, lung cancer, breast cancer, skin cancer, and prostate cancer; and known other cancers that affect the epithelial cells, mesenchymal cells, and blood cells in the whole body.

A fifth embodiment of the present invention relates to a method for preventing and/or treating cancer, comprising administering the pharmaceutical composition of the present invention to a patient (hereinafter also referred to as "the preventive or therapeutic method of the present invention").

The term "to treat" means herein to inhibit or alleviate progression and deterioration of the pathological condition of a patient who has already been affected with a cancer, and it means a treatment for the purpose of inhibiting or alleviating progression and deterioration of the cancer by doing so.

On the other hand, the term "to prevent" means herein to previously inhibit the onset of a cancer that needs to be treated, in a person who is likely to develop the cancer, and it is a treatment for the purpose of previously inhibit the onset of the cancer by doing so. Further, a treatment for inhibiting the recurrence of a cancer after completion of the cancer therapy is also included in the "prevention."

In addition, the therapeutic or preventive target is not limited to a human, and examples of the therapeutic or preventive target may include mammals other than humans, for example, mice, rats, dogs, cats, livestock animals such as bovines, horses or sheep, and primates such as monkeys, chimpanzees or gorillas. The therapeutic or preventive target is particularly preferably a human.

Moreover, another embodiment of the present invention relates to a method for diagnosing cancer, using the antibody of the present invention. The antibody of the present invention can specifically bind to a human CDCP1 molecule, and by labeling the antibody of the present invention with a fluorescent substance, a radioisotope, an enzymes, etc., tumor and cancer cells expressing human CDCP1 molecules, human CDCP1 molecules present in blood, or the fragments thereof, and the like can be detected. Examples of such a detection method may include an immunostaining method, a flow cytometry method, a Western blotting method, an ELISA method, a RIA method, a CLIA method, and a PET method. Cancer cells existing in a body can be directly detected, or the expression level of human CDCP1 in a patient specimen can be observed, so that the presence or absence of a primary tumor, the presence or absence of a metastatic tumor, etc. can be evaluated. Furthermore, the expression level of human CDCP1 in a cancer case has been previously diagnosed by a method using the antibody of the present invention, so that therapeutic effects obtained by administration of a pharmaceutical composition comprising the antibody of the present invention can be predicted.

The cancer as a diagnostic target may be any cancer, and is not particularly limited. Representative examples of the cancer may include: malignant tumors, such as hepatocellular carcinoma, bile duct cell carcinoma, renal cell carcinoma, squamous cell carcinoma, basal cell carcinoma, transitional cell carcinoma, adenocarcinoma, malignant gastrinoma, malignant melanoma, fibrosarcoma, myxosarcoma, liposarcoma, leiomyosarcoma, rhabdomyosarcoma, malignant teratoma, angiosarcoma, Kaposi's sarcoma, osteosarcoma, chondrosarcoma, lymphangiosarcoma, malignant meningioma, non-Hodgkin lymphoma, Hodgkin lymphoma, leukemia, and brain tumor; malignant neoplasms, such as epithelial cell-derived neoplasm (epithelial carcinoma), basal cell carcinomas, adenocarcinomas, lip cancer, oral cancer, esophageal cancer, gastrointestinal cancer such as small bowel cancer and stomach cancer, colon cancer, rectal cancer, bladder cancer, pancreatic cancer, ovarian cancer, cervical cancer, lung cancer, breast cancer, skin cancer, and prostate cancer; and known other cancers that affect the epithelial cells, mesenchymal cells, and blood cells in the whole body.

The disclosures of all publications cited in the present description are incorporated herein by reference in their entirety. In addition, throughout the present description, when singular terms with the articles "a," "an," and "the" are used, these terms include not only single items but also multiple items, unless otherwise clearly specified.

Hereinafter, the present invention will be further described in the following examples. However, these examples are only illustrative examples of the embodiments of the present invention, and thus, are not intended to limit the scope of the present invention.

### Examples

### 1. Expression and purification of recombinant hCDCP1 extracellular domain protein

The amino acid sequence of the hCDCP1 protein has been registered as Isoform 1 of UniProt Registration No. Q9H5V8 (SEQ ID NO: 1). With respect to the amino acids at positions 30 to 666 of this sequence, a Campath secretory signal was ligated to the N-terminal side thereof, and a 6 x His tag and a FLAG tag were inserted into the C-terminal side thereof, so as to design a sequence (SEQ ID NO: 2). This amino acid sequence was converted to a nucleotide sequence on the basis of a codon table for mammals, and a DNA sequence (SEQ ID NO: 3), in which a Kozak translational initiation sequence was inserted into the 5'-terminus thereof and a translation stop codon was inserted into the 3'-terminal side thereof, was synthesized according to gene synthesis (GENEWIZ). The synthesized DNA was inserted between the restriction enzyme KpnI recognition sequence and PmeI recognition sequence of pEF1/VS-His A (Thermo Scientific). The thus produced expression vector was used as follows.

The expression vector plasmid was transiently transfected into FreeStyle293 cells (Thermo Scientific) according to a polyethyleneimine method, and the cells were then cultured at 37°C in a 5% CO₂ incubator for 5 days. Thereafter, a culture supernatant was recovered and was then filtrated through a 0.22 µm filter, and the resultant was then allowed to bind to a HisTrap excel column (GE Healthcare). Subsequently, it was eluted with a concentration gradient of 20 mM to 500 mM imidazole in a 20 mM phosphate buffer/300 mM NaCl/pH 7.5 buffer. The elution fractions were separated by 1 mL, and a fraction that was observed to have a band of about 100 kDa according to an SDS-PAGE method was recovered.

A 9-fold amount of 20 mM Tris-HCl (pH 8.0) was added to this elution fraction, and the obtained mixture was allowed to bind to a HiTrap Q (GE Healthcare) column. Elution was carried out with a concentration gradient of 0 M to 1 M NaCl in a 20 mM Tris-HCl (pH 8.0) buffer. The elution fractions were separated by 2.5 mL, and a fraction whose band could be confirmed by the SDS-PAGE method was recovered.

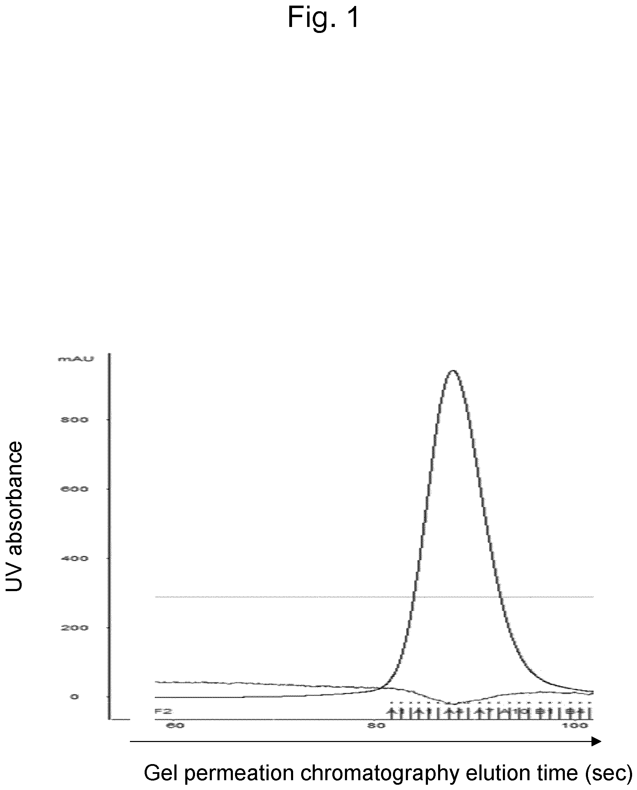

Furthermore, the elution fraction in this ion exchange column was concentrated to about 1.7 mL, and was then fractionated using D-PBS (Nacalai Tesque, Inc.) as a mobile phase, and employing Superdex 200 pg 16/60 (GE Healthcare). The elution curve in the purification experiment of using this gel permeation chromatography is shown in Figure 1. The elution fractions were separated by 1.0 mL, and a fraction whose band could be confirmed by the SDS-PAGE method was recovered. The recovered protein was used as a purified recombinant hCDCP1 extracellular domain protein (hereinafter referred to as an "hCDCP1-ECD protein").

### 2. Plasmin treatment and purification of full-length ECD protein

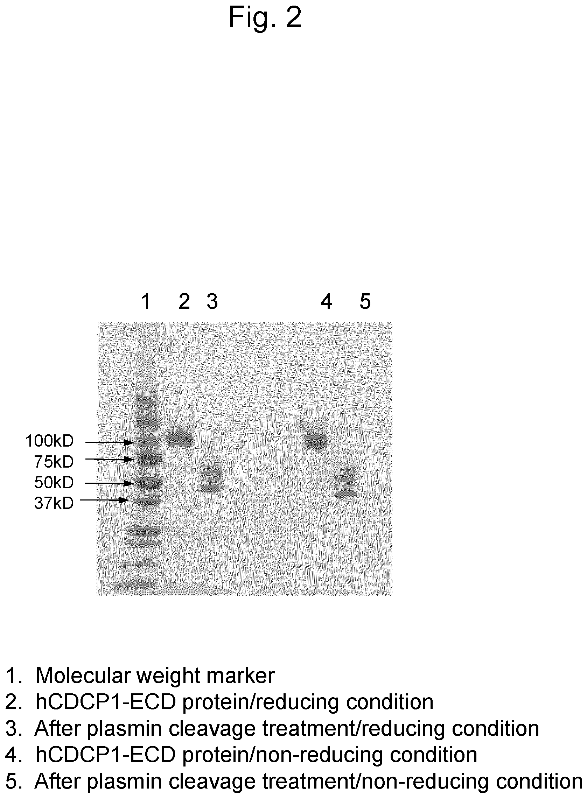

To the hCDCP1-ECD protein, 1/25 amount of human plasma-derived plasmin protein (Sigma-Aldrich) was added, and the obtained mixture was then reacted at 4°C for 18 hours. According to the SDS-PAGE method, it was confirmed that the hCDCP1-ECD protein was almost completely cleaved by this plasmin treatment. This protein sample was purified using cOmplete His-Tag purification resin (Sigma-Aldrich) and anti-FLAG M2 antibody affinity gel (Sigma-Aldrich), and the resulting sample was then observed by SDS-PAGE under reducing and non-reducing conditions. The results are shown in Figure 2. The eluted protein was considered to contain two molecular species having different molecular weights. These two molecular species were considered to be two peptide fragments resulting from the cleavage of hCDCP1-ECD, and it was considered that these two fragments were purified, while maintaining the bound state in some way, even after the cleavage.

### 3. Construction of full-length hCDCP1 stable expression Ba/F3 cells

A sequence (SEQ ID NO: 4) was designed by ligating a Campath secretory signal and a c-Myc tag to the N-terminal side of the amino acids at positions 30 to 836 of Isoform 1 of UniProt Registration No. Q9H5V8. This amino acid sequence was converted to a nucleotide sequence on the basis of a codon table for mammals, and DNA comprising this sequence and having a Kozak sequence at the 5'-terminus and a translation stop codon at the 3'-terminus inserted was synthesized according to gene synthesis (GENEWIZ; SEQ ID NO: 5). The synthesized DNA was inserted between the KpnI/PmeI recognition sequences of pEF1 / V5-His A (Thermo Scientific) to produce the expression vector GS01.

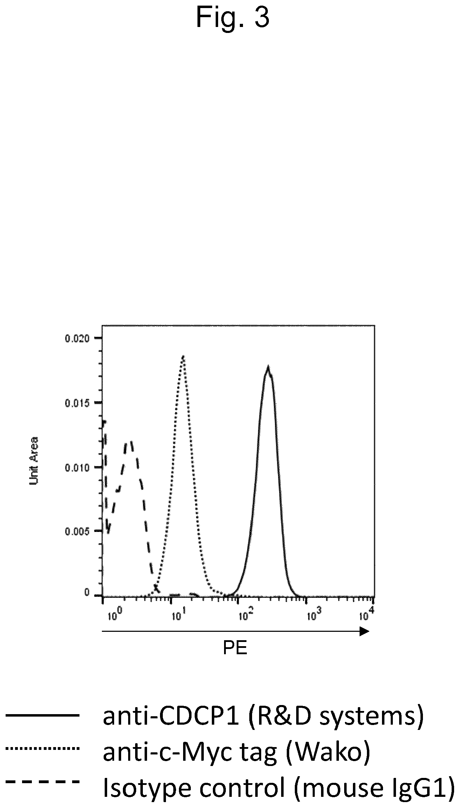

The plasmid GS01 was linearized with the restriction enzyme PmeI, and 2 µg thereof was then introduced into mouse B cell-derived cell line Ba/F3 (2 × 10⁶ cells) by using Nucleofector 2b (Lonza). After the gene introduction, the cells were seeded in a 96-well plate, and G418 (Nacalai Tesque, Inc.) was then added thereto to a final concentration of 1 µg/mL. Six days later, 24 colonies, in which G418-resistant proliferation was observed, were recovered from the wells, and the expression of hCDCP1 was then confirmed by flow cytometry using an anti-hCDCP1 antibody (R&D systems; Catalog No. MAB26662). Then, limitng dilution was carried out on cells derived from the thus confirmed colonies. Proliferating cells were recovered, and the expression of hCDCP1 was then confirmed by flow cytometry using an anti-hCDCP1 antibody (R&D systems), and finally, a cell line derived from monoclones was selected. These cells were stained with the same above anti-hCDCP1 antibody, an anti-Myc tag antibody (Wako Pure Chemical Industries, Ltd.) and a PE-labeled anti-mouse IgG secondary antibody. The results are shown in Figure 3. It was confirmed that all clones were positive to the anti-hCDCP1 antibody and the anti-Myc tag antibody, and that a product of the hCDCP1 gene with the Myc tag added to the gene-introduced N-terminus was expressed on the cell surface. These cells were used as a Ba/F3-hCDCP1 cell line in the subsequent experiments.

### 4. Generation of ΔN-type molecule by trypsin treatment on Ba/F3-hCDCP1

The hCDCP1-expressing Ba/F3 cell line (hereinafter referred to as "Ba/F3-hCDCP1") prepared in the previous section and a Ba/F3 original cell line as a negative target were used. The cells were washed twice with a cold RPMI1640 medium (Sigma-Aldrich) contained no serum, and were then suspended in an RPMI1640 medium at a concentration of 1.25 × 10⁷ cells/mL. To 800 µL of the cell suspension, 200 µL of 0.25% trypsin/EDTA (Nacalai Tesque, Inc.), or Hank's Balanced Salt Solution (hereinafter referred to as "HBSS") containing 1 mM EDTA used as a negative control, was added, and the obtained mixture was then treated at 37°C for 0, 5, 15, and 30 minutes. After completion of the reaction, 9 mL of an RPMI1640 medium supplemented with 10% heat inactivated fetal bovine serum (hereinafter referred to as "FBS") was added to the reaction mixture to terminate the reaction. After these cells were washed with PBS containing 1% BSA (hereinafter referred to as a "flow cytometry buffer" or "FCM buffer"), the cells were stained with an anti-Myc tag antibody (Wako Pure Chemical Industries, Ltd.) and an anti-hCDCP1 antibody (R&D systems). The cells were washed and were then stained with a PE-labeled anti-mouse IgG antibody (Becton Dickinson). After washing the cells, the cells were observed with FACSCantoII (Becton Dickinson).

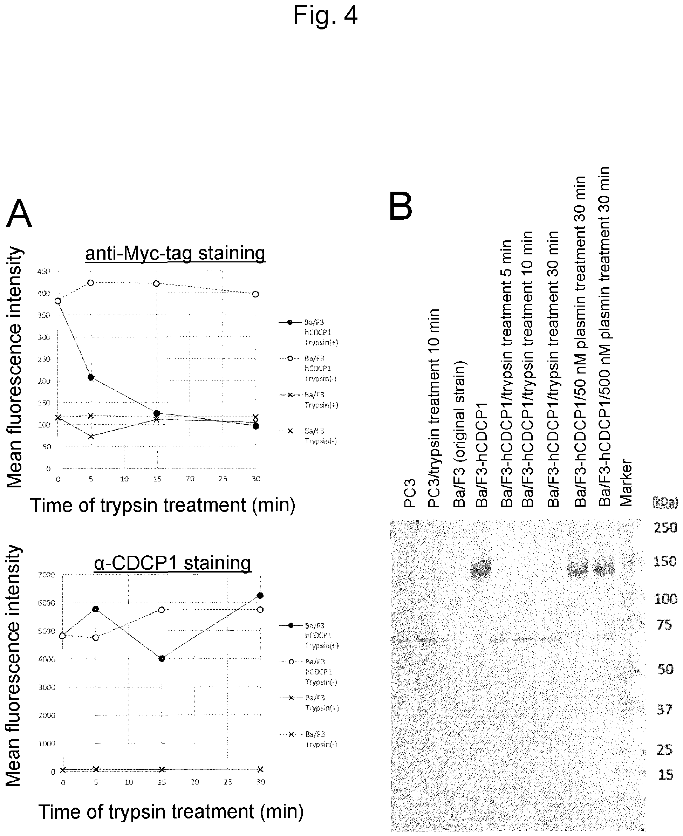

Figure 4A is a graph showing a change in the mean fluorescence intensity of the cell population from the fluorescence intensity of PE measured by flow cytometry. The staining intensity of the anti-Myc tag antibody decreased over time, only when trypsin/EDTA was added. These results show that the N-termini of the hCDCP1 molecules on Ba/F3-hCDCP1 were digested by trypsin. On the other hand, the staining intensity of the anti-hCDCP1 antibody did not change, and these results show that the number of hCDCP1 molecules recognized by the anti-hCDCP1 antibody used in the present experiment was not reduced even by the trypsin treatment.

Subsequently, the molecular weight of the cleaved hCDCP1 molecules produced by the trypsin treatment was observed by Western blotting. Ba/F3-hCDCP1 cells (4 × 10⁶ cells) were treated with 0.25% trypsin/EDTA diluted 5-fold with an RPMI1640 medium or with PBS containing 50 nM or 500 nM plasmin (Sigma-Aldrich) at 37°C for 5 minutes, 10 minutes, or 30 minutes. Thereafter, these cells were recovered and were then washed with HBSS, and the resulting cells were dissolved in a Tris buffered saline containing 1% TritonX and a protease inhibitor cocktail (Thermo Fisher Scientific). After that, protein quantification was carried out using Micro BCA kit (Thermo Fisher Scientific), and 20 µg of the protein was then electrophoresed according to SDS-PAGE. At the same time, the human prostate cancer-derived cell line PC3 (ATCC), which has been known to express cleaved hCDCP1, was also cytolyzed in an adherent culture state, or was treated with a diluted trypsin/EDTA solution, as with Ba/F3, and the resulting cells were then cytolyzed. The resultant was also subjected to SDS-PAGE. A protein was transferred from the electrophoresed gel onto a PVDF membrane, and Western blotting was then performed using an anti-hCDCP1 intracellular domain antibody (Abcam) as a primary antibody for detection, also using an alkaline phosphatase-labeled anti-goat IgG antibody (Promega) as a secondary antibody, and also using an NBT/BCIP stock solution (Roche) NBT as a detection reagent.

The results of the Western blotting are shown in Figure 4B. In the Ba/F3-hCDCP1 cells without trypsin treatments, an almost a single band was observed around 130 kDa. In contrast, in the trypsin treated cells, a single band was observed around 70 kDa, and this band size was considered to be at the same position as the band detected from the lysed sample of PC3 cells. Thus, it was suggested that the molecular species generated by the trypsin treatment of Ba/F3 should be the same as the cleaved hCDCP1 expressed by PC3. A band of a cleaved protein was detected at the same mobility also by the treatment with 500 nM plasmin.

Thereby, it was suggested that hCDCP1 expressed by Ba/F3-CDCP1 should be cleaved by treatments with trypsin and plasmin, and that the cleaved hCDCP1 molecules should remain on the cells. Subsequently, by using this technique, antibodies that react with the cleaved hCDCP1 molecules were screened.

### 5. Screening of hybridomas using hCDCP1 knockout cell line

Hybridomas were prepared using the proteins and cells prepared in the previous sections, and thereafter, hybridomas producing antibodies exhibiting hCDCP1-specific reactions were screened.

PC3 cells, in which hCDCP1 molecules were knocked out, were produced, and were then used as negative controls in the screening for clones that would react with hCDCP1 expressed by cancer cells. The used human prostate cancer cell line PC3 was purchased from ATCC. CDCP1 CRISPR/Cas9 KO plasmid (h) (SantaCruz) was introduced into 1 × 10⁶ PC3 cells, using Nucleofector 2b (Lonza). This plasmid contained the GFP gene sequence. One day later, GFP fluorescence-positive cells were single-cell-sorted into a 96-well culture plate, using FACSAriaII (Becton Dickinson). Seven to ten days after the single cell sorting, clones, in which cell proliferation was observed, were subjected to expression confirmation by flow cytometry using an anti-hCDCP1 antibody (R&D systems), and cell clones that did not express hCDCP1 were then proliferated. The thus proliferated cell clones were used as "hCDCP1-KO PC3 cells" in the subsequent experiments.

Monoclonal antibodies reacting against hCDCP1 were prepared, using lymph node cells that had been isolated from mice immunized with hCDCP1 purified proteins or hCDCP1 forced expression cells.

In Experiment 1, 50 µg of an hCDCP1-ECD protein that was mixed with TiterMaxGold (TiterMax) was administered per mouse via intravenous administration into the sole of the foot. Seven days and ten days after the intravenous administration, 10 µg of the antigen mixed with TiterMaxGold was administered per mouse as a booster via intravenous administration into the sole of the foot.

In Experiment 2, Ba/F3-hCDCP1 cells were used as immune substances. After the cells had been treated with trypsin by the aforementioned method, 1 × 10⁷ cells per mouse were mixed with TiterMaxGold (TiterMax), and the obtained mixture was then intravenously administered into the sole of the foot. Seven days and ten days after the intravenous administration, 1 × 10⁵ trypsin-treated Ba/F3-hCDCP1 cells suspended in PBS were intraperitoneally administered per mouse as a booster.

In Experiment 3, an hCDCP1-ECD protein was cleaved with plasmin according to the method described in the previous section, and was then purified using a His-tag and a FLAG-tag affinity resin. Thereafter, 50 µg of the resultant that was mixed with TiterMaxGold was administered per mouse via intravenous administration into the sole of the foot. Seven days and ten days after the intravenous administration, 10 µg of the antigen mixed with TiterMaxGold was administered per mouse as a booster via intravenous administration into the sole of the foot.

Regarding each of Experiment 1, Experiment 2 and Experiment 3, popliteal lymph nodes were collected from mice after completion of the immunization, and cell suspensions were then prepared. The resulting cells were mixed with SP2/0-Ag14 myeloma cells in a serum-free RPMI1640 (ATCC modified; Thermo Fisher Scientific), and cell fusion was carried out using polyethylene glycol (Roche). The fused cells were suspended in ClonaCell TM-HY Medium D (STEMCELL) containing Hybridoma enhancing medium (Sigma-Aldrich), and the obtained suspension was then seeded in a plastic petri dish. Colonies formed 8 to 10 days after the seeding were isolated in a 96-well plastic plate, in which a medium (RPMI1640/10% FBS/HAT supplement (Thermo Fisher Scientific)/Hybridoma enhancing medium (Sigma-Aldrich)) was dispensed, followed by performing an expansion culture. Thereafter, the culture supernatant was used in the evaluation of binding property.

According to a Cell-ELISA method using PC3 cells and hCDCP1-KO PC3 cells, the hCDCP1 binding property of the antibody produced by the hybridoma was screened. PC3 cells and hCDCP1-KO PC3 cells were seeded in a 384-well plate at a cell density of 5000 cells/well, and were then cultured at 37°C overnight. After the removal of the culture medium, 25 µL per well of the hybridoma culture supernatant or the antibody solution diluted with a medium was added, and the obtained mixture was then reacted at 4°C for 1 hour. Thereafter, the culture supernatant was removed, and the residue was then washed with PBS. A secondary medium was diluted with a medium and was then added, and the obtained mixture was reacted at 4°C for 1 hour. Thereafter, the reaction mixture was further washed with PBS three times, and the washing solution was completely removed. Thereafter, the substrate solution (ELISA POD substrate TMB Kit, Nacalai Tesque, Inc.) was reacted with the residue, and 10 minutes after initiation of the reaction, the reaction was terminated with 1 N sulfuric acid. Then, the absorbance at 450 nm was measured.

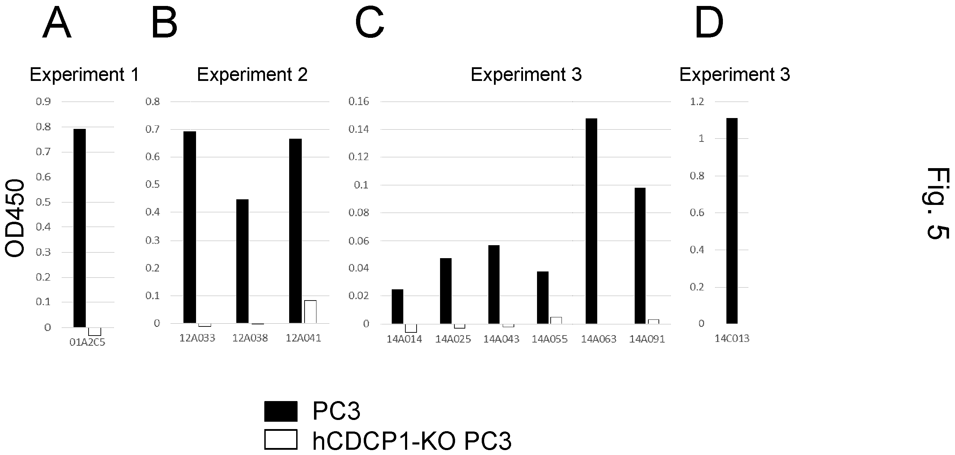

Cell-ELISA was performed using culture supernatants of the hybridomas produced from individual immune animals used in Experiment 1, Experiment 2, and Experiment 3. The results are shown in Figures 5A, 5B, 5C, and 5D. The numerical values shown in the figures indicate numerical values obtained by subtracting the values obtained by reacting with the hybridoma culture medium containing no antibody, from the measured values of individual clones. From these results, it was demonstrated that hybridomas, which produced antibodies that react with PC3 cells as cancer cells expressing hCDCP1, were obtained from immune mice using hCDCP1 purified proteins or hCDCP1 forced expression cells. In addition, from the results of Figure 5A, Figure 5B and Figure 5C, it was demonstrated that the antibodies produced from the hybridomas shown in these figures did not react with hCDCP1-KO PC3 cells, and that these antibodies certainly specifically reacted with hCDCP1 expressed by PC3.

### 6. Antibody sequence analysis

Regarding the hybridomas that produce antibodies that specifically react with hCDCP1 expressed by PC3 cells, as described in the previous section, the sequences of the immunoglobulin gene variable regions thereof were analyzed as follows.

The hybridoma cells were subjected to an expansion culture, and RNA extraction and cDNA synthesis using reverse transcriptase were then performed using SuperPrep II Cell Lysis & RT Kit (Toyobo Co., Ltd.). The antibody genes were amplified from the synthesized cDNA according to a PCR method. Both the heavy and the light chains were amplified, using primers that recognized sites upstream of the variable regions and sites downstream of the constant regions. The primer sequences used are as follows.

For amplification of heavy chain 5': 5'MsVHE (SEQ ID NO: 6)

For amplification of heavy chain 3': 3'Cg1_outer (SEQ ID NO: 7), 3'Cg2c_outer (SEQ ID NO: 8), 3'Cg2b_outer (SEQ ID NO: 9), 3'Cg3_outer (SEQ ID NO: 10), and 3'mIgG2a_CH (SEQ ID NO: 11)

For amplification of _{K} light chain 5': 5'L-Vk_3 (SEQ ID NO: 12), 5'L-Vk_4 (SEQ ID NO: 13), 5'L-Vk_5 (SEQ ID NO: 14), 5'L-Vk_6 (SEQ ID NO: 15), 5'L-Vk_689 (SEQ ID NO: 16), 5'L-Vk_14 (SEQ ID NO: 17), 5'L-Vk_19 (SEQ ID NO: 18), and 5'L-Vk_20 (SEQ ID NO: 19)

For amplification of κ light chain 3': 3'mCk (SEQ ID NO: 20)

For amplification of λ light chain 5': 5'mVλ1/2 (SEQ ID NO: 21) and 5'mVλx (SEQ ID NO: 22)

For amplification of λ light chain 3': 3'mCλ_outer (SEQ ID NO: 23)

The obtained DNA fragments were each cloned into a pcDNA3.4 vector by using TOPO TA cloning kit (Thermo Scientific), and the DNA sequences were then analyzed. Regarding the antibody sequences obtained as a result of the analysis, CDR sequences were determined according to the method of Kabat et al. (Sequences of Proteins of Immunological Interests, Fifth edition, NIH Publication No. 91-3242, US Department of Health and Human Services, 1991). The antibody sequences of the analyzed clones and their CDR region sequences are summarized in Table 1 below.

**[Table 1]**

| | VH | CDRH1 | CDRH2 | CDRH3 | VL | CDRL1 | CDRL2 | CDRL3 |
|---|---|---|---|---|---|---|---|---|
| 01A2C5 | SEQ ID NO: 24 | SEQ ID NO: 25 | SEQ ID NO: 26 | SEQ ID NO: 27 | SEQ ID NO: 28 | SEQ ID NO: 29 | SEQ ID NO: 30 | SEQ ID NO: 31 |
| 12A033 | SEQ ID NO: 32 | SEQ ID NO: 33 | SEQ ID NO: 34 | SEQ ID NO: 35 | SEQ ID NO: 36 | SEQ ID NO: 37 | SEQ ID NO: 38 | SEQ ID NO: 39 |
| 12A038 | SEQ ID NO: 40 | SEQ ID NO: 41 | SEQ ID NO: 42 | SEQ ID NO: 43 | SEQ ID NO: 44 | SEQ ID NO: 45 | SEQ ID NO: 46 | SEQ ID NO: 47 |
| 12A041 | SEQ ID NO: 48 | SEQ ID NO: 49 | SEQ ID NO: 50 | SEQ ID NO: 51 | SEQ ID NO: 52 | SEQ ID NO: 53 | SEQ ID NO: 54 | SEQ ID NO: 55 |
| 14A014 | SEQ ID NO: 56 | SEQ ID NO: 57 | SEQ ID NO: 58 | SEQ ID NO: 59 | SEQ ID NO: 60 | SEQ ID NO: 61 | SEQ ID NO: 62 | SEQ ID NO: 63 |
| 14A025 | SEQ ID NO: 64 | SEQ ID NO: 65 | SEQ ID NO: 66 | SEQ ID NO: 67 | SEQ ID NO: 68 | SEQ ID NO: 69 | SEQ ID NO: 70 | SEQ ID NO: 71 |
| 14A043 | SEQ ID NO: 72 | SEQ ID NO: 73 | SEQ ID NO: 74 | SEQ ID NO: 75 | SEQ ID NO: 76 | SEQ ID NO: 77 | SEQ ID NO: 78 | SEQ ID NO: 79 |
| 14A055 | SEQ ID NO: 80 | SEQ ID NO: 81 | SEQ ID NO: 82 | SEQ ID NO: 83 | SEQ ID NO: 84 | SEQ ID NO: 85 | SEQ ID NO: 86 | SEQ ID NO: 87 |
| 14A063 | SEQ ID NO: 88 | SEQ ID NO: 89 | SEQ ID NO: 90 | SEQ ID NO: 91 | SEQ ID NO: 92 | SEQ ID NO: 93 | SEQ ID NO: 94 | SEQ ID NO: 95 |
| 14A091 | SEQ ID NO: 96 | SEQ ID NO: 97 | SEQ ID NO: 98 | SEQ ID NO: 99 | SEQ ID NO: 100 | SEQ ID NO: 101 | SEQ ID NO: 102 | SEQ ID NO: 103 |
| 14C013 | SEQ ID NO: 104 | SEQ ID NO: 105 | SEQ ID NO: 106 | SEQ ID NO: 107 | SEQ ID NO: 108 | SEQ ID NO: 109 | SEQ ID NO: 110 | SEQ ID NO: 111 |

### 7. Preparation of mouse-human chimeric antibodies

Based on the antibody sequences obtained in the previous section, a chimeric antibody comprising mouse heavy chain/light chain variable regions and human IgG1 heavy chain/κ light chain constant regions (hereinafter referred to as a "mouse-human chimeric antibody") was prepared. Mouse-human chimeric antibodies were prepared based on the antibody sequences of 12A041, 14A025, 14A043, 14A055, 14A063 and 14A091, these 6 antibodies are selected among the antibodies whose sequences were analyzed in the previous section.

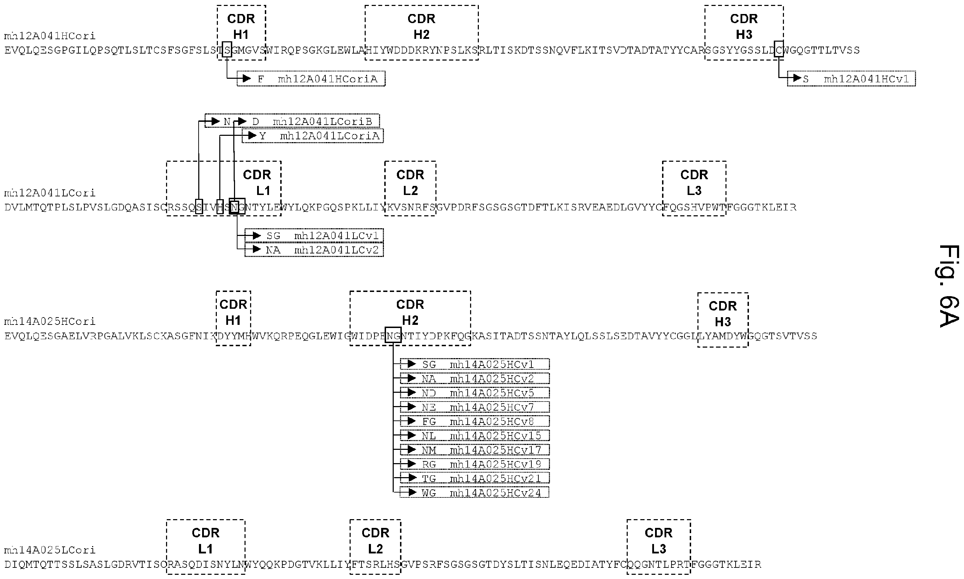

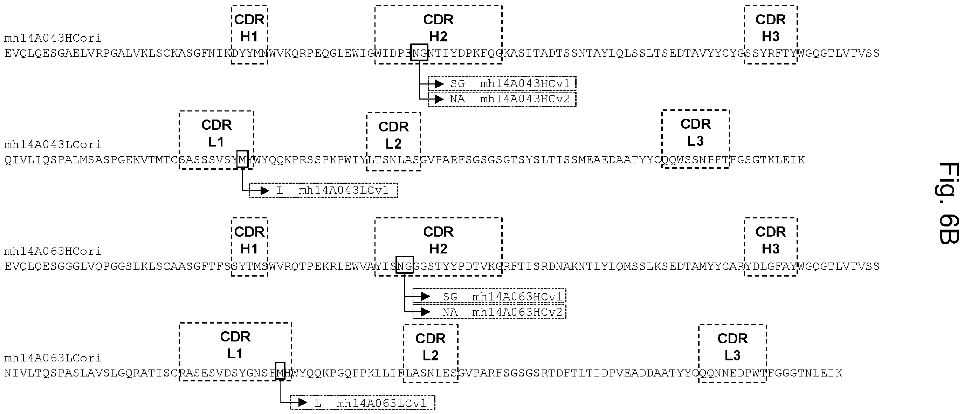

Regarding the sequences of the antibodies 12A041, 14A025, 14A043 and 14A063, the same amino acid sequences as those analyzed in the previous section were prepared, and at the same time, amino acid modification of the CDR regions was attempted. The correspondence among the introduction sites of sequence modifications, the modified amino acids, and the names of the modified antibody sequences is as shown in Figure 6A and Figure 6B, and modification was performed on cysteine residues existing in the CDR regions that did not seem to form an SS bond, and sequences that are highly likely to undergo cleavage and/or oxidation reactions of proteins in the solvent.

Moreover, regarding mh12A041HCori, the serine residue of Kabat No. 32 was mutated to a phenylalanine residue, and regarding mh12A041LCori, the histidine residue of Kabat No. 27d was mutated to a tyrosine residue, or the serine residue of Kabat No. 27a and the asparagine residue of Kabat No. 28 were simultaneously mutated to an asparagine residue and an aspartic acid residue, respectively, so that the affinity to an antigen seemed to be improved Thus, we designed sequences comprising these mutations (mh12A041HCoriA, mh12A041LCoriA, and mh12A041LCoriB).

Antibody sequences subjected to modification of CDR sequences, and sequences that are bases of such modified antibody sequences are shown in the following Table 2.

**[Table 2]**

| | Variable region sequence | CDR1 | CDR2 | CDR3 |
|---|---|---|---|---|
| mh12A041HCori | SEQ ID NO: 48 | SEQ ID NO: 49 | SEQ ID NO: 50 | SEQ ID NO: 51 |
| mh12A041HCv1 | SEQ ID NO: 112 | SEQ ID NO: 49 | SEQ ID NO: 50 | SEQ ID NO: 113 |
| mh12A041HCoriA | SEQ ID NO: 114 | SEQ ID NO: 115 | SEQ ID NO: 50 | SEQ ID NO: 51 |
| mh12A041LCori | SEQ ID NO: 52 | SEQ ID NO: 53 | SEQ ID NO: 54 | SEQ ID NO: 55 |
| mh12A041LCv1 | SEQ ID NO: 116 | SEQ ID NO: 117 | SEQ ID NO: 54 | SEQ ID NO: 55 |
| mh12A041LCv2 | SEQ ID NO: 118 | SEQ ID NO: 119 | SEQ ID NO: 54 | SEQ ID NO: 55 |
| mh12A041LCoriA | SEQ ID NO: 120 | SEQ ID NO: 121 | SEQ ID NO: 54 | SEQ ID NO: 55 |
| mh12A041LCoriB | SEQ ID NO: 122 | SEQ ID NO: 123 | SEQ ID NO: 54 | SEQ ID NO: 55 |
| mh14A025HCori | SEQ ID NO: 64 | SEQ ID NO: 65 | SEQ ID NO: 66 | SEQ ID NO: 67 |
| mh14A025HCv1 | SEQ ID NO: 124 | SEQ ID NO: 65 | SEQ ID NO: 125 | SEQ ID NO: 67 |
| mh14A025HCv2 | SEQ ID NO: 126 | SEQ ID NO: 65 | SEQ ID NO: 127 | SEQ ID NO: 67 |
| mh14A025HCv5 | SEQ ID NO: 128 | SEQ ID NO: 65 | SEQ ID NO: 129 | SEQ ID NO: 67 |
| mh14A025HCv7 | SEQ ID NO: 130 | SEQ ID NO: 65 | SEQ ID NO: 131 | SEQ ID NO: 67 |
| mh14A025HCv8 | SEQ ID NO: 132 | SEQ ID NO: 65 | SEQ ID NO: 133 | SEQ ID NO: 67 |
| mh14A025HCv5 | SEQ ID NO: 134 | SEQ ID NO: 65 | SEQ ID NO: 135 | SEQ ID NO: 67 |
| mh14A025HCv7 | SEQ ID NO: 136 | SEQ ID NO: 65 | SEQ ID NO: 137 | SEQ ID NO: 67 |
| mh14A025HCv19 | SEQ ID NO: 138 | SEQ ID NO: 65 | SEQ ID NO: 139 | SEQ ID NO: 67 |
| mh14A025HCv21 | SEQ ID NO: 140 | SEQ ID NO: 65 | SEQ ID NO: 141 | SEQ ID NO: 67 |
| mh14A025HCv24 | SEQ ID NO: 142 | SEQ ID NO: 65 | SEQ ID NO: 143 | SEQ ID NO: 67 |
| mh14A025LCori | SEQ ID NO: 68 | SEQ ID NO: 69 | SEQ ID NO: 70 | SEQ ID NO: 71 |
| mh14A043HCori | SEQ ID NO: 72 | SEQ ID NO: 73 | SEQ ID NO: 74 | SEQ ID NO: 75 |
| mh14A043HCv1 | SEQ ID NO: 144 | SEQ ID NO: 73 | SEQ ID NO: 145 | SEQ ID NO: 75 |
| mh14A043HCv2 | SEQ ID NO: 146 | SEQ ID NO: 73 | SEQ ID NO: 147 | SEQ ID NO: 75 |
| mh14A043LCori | SEQ ID NO: 76 | SEQ ID NO: 77 | SEQ ID NO: 78 | SEQ ID NO: 79 |
| mh14A043LCv1 | SEQ ID NO: 148 | SEQ ID NO: 149 | SEQ ID NO: 78 | SEQ ID NO: 79 |
| mh14A063HCori | SEQ ID NO: 88 | SEQ ID NO: 89 | SEQ ID NO: 90 | SEQ ID NO: 91 |
| mh14A063HCv1 | SEQ ID NO: 150 | SEQ ID NO: 89 | SEQ ID NO: 151 | SEQ ID NO: 91 |
| mh14A063HCv2 | SEQ ID NO: 152 | SEQ ID NO: 89 | SEQ ID NO: 153 | SEQ ID NO: 91 |
| mh14A063LCori | SEQ ID NO: 154 | SEQ ID NO: 93 | SEQ ID NO: 94 | SEQ ID NO: 95 |
| mh14A063LCv1 | SEQ ID NO: 155 | SEQ ID NO: 156 | SEQ ID NO: 94 | SEQ ID NO: 95 |

Regarding the sequences shown in Table 2, nucleotide sequences were designed based on the amino acid sequences of the antibody molecules, and an expression vector was synthesized by gene synthesis.

In addition, regarding the 14A055 heavy chain variable region (SEQ ID NO: 80), the 14A055 light chain variable region (SEQ ID NO: 84), the 14A091 heavy chain variable region (SEQ ID NO: 96), and the 14A091 light chain variable region (SEQ ID NO: 100), an expression vector was produced by amplifying according to a PCR method, variable region DNA from the sequence subcloned into a pcDNA3.4 vector in the previous section, followed by subcloning it.

With regard to vectors used, a pFUSE-CHIg-hG1 vector (Invitrogen) was used for the cloning of the heavy chain variable region sequences of antibody groups other than the mh12A041 antibody group and the mh14A025 antibody group; and a pFUSE-CHIOME-HC vector was used for the cloning of the heavy chain variable region sequences of the mh12A041 antibody group and the mh14A025 antibody group. For the cloning of the light chain variable region sequences of all antibody groups, a pFUSE2-CLIg-hk vector (Invitrogen) was used. The amino acid sequences of the antibody constant regions possessed by these three vectors are shown in SEQ ID NO: 157, SEQ ID NO: 158, and SEQ ID NO: 159, respectively.

The prepared expression vectors were allowed to express in Expi293 cells (Thermo Fisher Scientific). At this time, regarding individual antibody groups mh12A041, mh14A025, mh14A043, and mh14A063, heavy chain and light chain vectors were combined in the following patterns and were then allowed to express.

### mh12A041:

HCori/LCori, HCori/LCv1, HCori/LCv2, HCv1/LCori, HCv1/LCv1, HCv1/LCv2, HCori/LCoriA, HCori/LCoriB, HCoriA/LCori, HCoriA/LCoriA, and HCoriA/LCoriB mh14A025: HCori/LCori, HCv1/LCori, HCv2/LCori, HCv5/LCori, HCv7/LCori, HCv8/LCori, HCv15/LCori, HCv17/LCori, HCv19/LCori, HCv21/LCori, and HCv24/LCori mh14A043:
HCori/LCori, HCori/LCv1, HCv1/LCori, HCv1/LCv1, HCv2/LCori, and HCv2/LCv1 mh14A063:
HCori/LCori, HCori/LCv1, HCv1/LCori, HCv1/LCv1, HCv2/LCori, and HCv2/LCv1

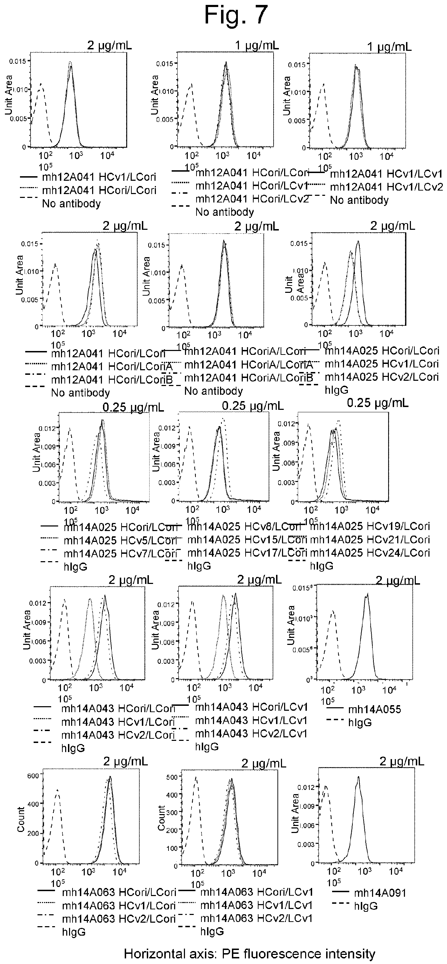

The concentration of an antibody protein secreted into a culture supernatant was measured according to an AlphaLISA method, and thereafter, the antibody protein was diluted to each concentration based on the measurement value and was then allowed to bind to Ba/F3-hCDCP1 cells. Further, a PE-labeled anti-human antibody (Becton Dickinson) was reacted as a secondary antibody with the cells, and the fluorescence of PE was then measured by flow cytometry, so that the reactivity of each antibody was evaluated. The results are shown in Figure 7. As shown in the figure, all of the evaluated antibodies retained reactivity to hCDCP1.

### 8. Reactivity of antibodies to trypsin-treated hCDCP1 forced expression cells

In order to investigate whether the obtained anti-hCDCP1 mouse-human chimeric antibodies bind to the N-terminal side of the serine protease cleavage site of the hCDCP1 molecules or bind to the transmembrane region side, the reactivity of the antibodies to trypsin-treated hCDCP1 forced expression Ba/F3 cells was verified according to the technique mentioned in the previous section. The following anti-hCDCP1 mouse-human chimeric antibody clones were evaluated: mh12A041HCori/LCori, mh14A025HCori/LCori, mh14A043HCori/LCori, mh14A055, mh14A063HCori/LCori, and mh14A091.

Ba/F3-hCDCP1 cells were treated in an RPMI1640 medium containing 0.05% trypsin at 37°C for 30 minutes, and were then washed twice with RPMI1640/10% FBS. It was confirmed by the same Western blotting as that mentioned in the previous section that the full-length hCDCP1 disappeared and only the cleaved hCDCP1 was present in the above-treated Ba/F3-CDCP1 cells. With respect to these cells, a dilution series of anti-hCDCP1 mouse-human chimeric antibodies from 5 µg/mL to 5 ng/mL was prepared, and was then reacted at 4°C for 30 minutes. As a negative control, a similar dilution series of anti-RS virus antibodies described in the later section was prepared as a non-specific human IgG, and was then reacted at 4°C for 30 minutes. Subsequently, a PE-labeled anti-human IgG Fc antibody (Southern Biotech) was allowed to act as a secondary antibody on the resulting cells, and after the resultant had been washed, the fluorescence intensity was observed with FACSCantoII (BECTON DICKINSON).

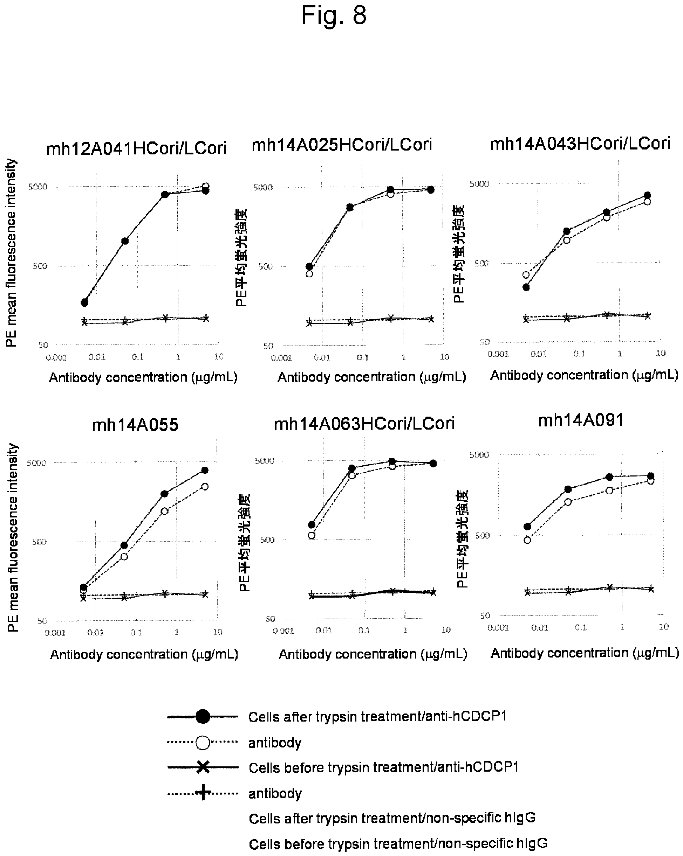

The results are shown in Figure 8. From these results, it was demonstrated that all of the evaluated six antibodies reacted with cells having hCDCP1 molecules that were cleaved by the trypsin treatment at the same level as in the case of not treating the cells with trypsin, and thus that these antibodies bound to the transmembrane region side of hCDCP1 molecules, rather than to the serine protease cleavage site thereof.

### 9. Reactivity of antibodies to crab-eating macaque CDCP1 molecules

A sequence (SEQ ID NO: 161) was designed by ligating a Campath secretory signal and a c-Myc tag to the N-terminal side of the amino acids at positions 30 to 836 of the CDCP1 protein (isoform X1; NCBI Reference Sequence: XP_005546930.1; SEQ ID NO: 160) of a crab-eating macaque (Macaca fascicularis). This amino acid sequence was converted to a nucleotide sequence based on a codon table for mammals, and DNA comprising this sequence and having a Kozak translational initiation sequence at the 5'-terminus and a translation stop codon at the 3'-terminus inserted was synthesized according to gene synthesis (GENEWIZ; SEQ ID NO: 162). The synthesized DNA was connected with the KpnI/Pmel site of pEF1/V5-His A (Thermo Fisher Scientific) to produce the expression vector GS02.

The plasmids GS01 (prepared in the above section) and GS02 were linearized with the restriction enzyme PmeI, and 2 µg of each plasmid was introduced into 2 × 10⁶ CHO-K1 cells by using Nucleofector (Lonza). After completion of the gene introduction operations, the cells were cultured in the presence of 400 µg/mL hygromycin for 3 days. Thereafter, the cells were exfoliated and dispersed, and were then stained with an anti-hCDCP1 antibody (R&D Systems). The positive cells were subjected to single-cell sorting using FACS AriaII (Becton Dickinson). After separation, proliferating cell clones were further stained with an anti-hCDCP1 antibody (R&D Systems), and clones expressing hCDCP1 and crab-eating macaque CDCP1 were thereby isolated. These clones were expanded, and were hereafter used as hCDCP1-expressing CHO-K1 cells and crab-eating macaque CDCP1-expressing CHO-K1 cells.

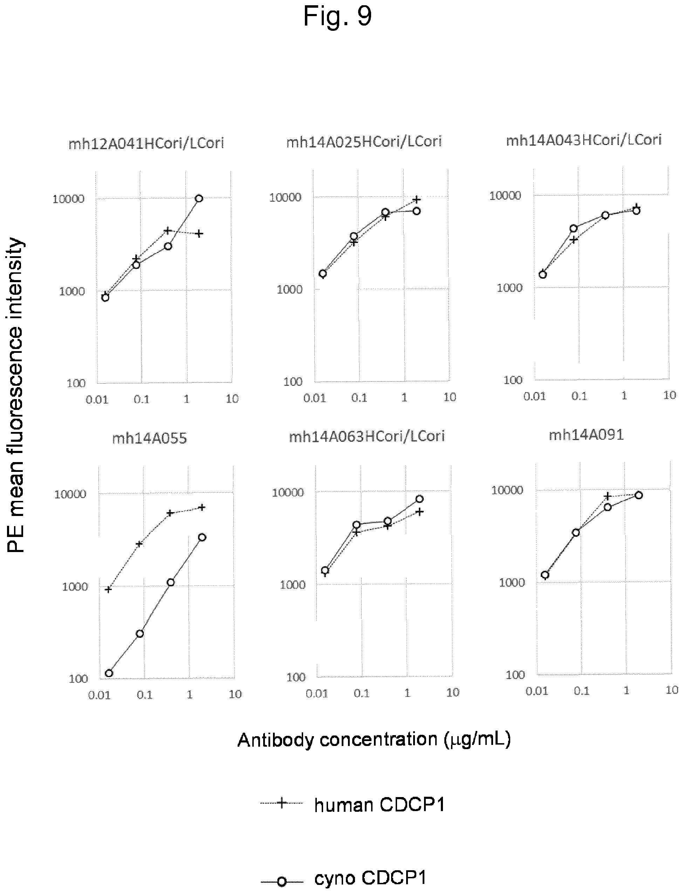

The hCDCP1-expressing CHO-K1 cells and the crab-eating macaque CDCP1-expressing CHO-K1 cells were exfoliated and dispersed using 0.25% trypsin/EDTA (Nacalai Tesque, Inc.), and were then reacted with various concentrations of individual mouse-human chimeric antibodies, namely, mh12A041HCori/LCori, mh14A025HCori/LCori, mh14A043HCori/LCori, mh14A055, mh14A063HCori/LCori, and mh14A091. Thereafter, the binding property of the antibodies was observed by flow cytometry (Figure 9). As a result, these 6 antibodies all reacted with the crab-eating macaque CDCP1, and in particular, the 5 antibodies other than mh14A055 reacted with both the human CDCP1 and the crab-eating macaque CDCP1 at the same levels.

### 10. Reactivity of antibodies to human cancer cells

In order to investigate the reactivity of the anti-hCDCP1 antibody group to individual human cancer cell lines, the binding reactivity of the antibodies was evaluated by flow cytometry. The human cancer cell lines used and their sources are as follows.
Prostate cancer: PC3 (ATCC), DU145 (RIKEN BioResource Center)
Lung cancer: H358 (ATCC), SK-MES-1 (ATCC)
Breast cancer: MDA-MB-231 (ATCC), HCC1143 (ATCC)
Bile duct cancer: TFK-1 (RIKEN BioResource Center)
Ovarian cancer: SK-OV3 (ATCC), OVCAR3 (ATCC)
Pancreatic cancer: Capan-2 (ATCC)
Colorectal/rectal cancer: DLD-1 (JCRB)
Normal cells: Human mammary epithelial cells (hereinafter referred to as "HMEpC") (Cell Applications), normal human epidermal keratinocytes (hereinafter referred to as "NHEK") (PromoCell)

Individual human cancer cell lines and normal tissue-derived cells were cultured using a 10 cm petri dish, in a culture medium suitable for individual cells. The cells in the petri dish were exfoliated and suspended by a 0.25% trypsin/EDTA (Nacalai Tesque, Inc.) treatment, and were then washed with an FCM buffer. Thereafter, individual antibodies that had been diluted with FCM buffers to individual concentrations shown in the figure were added to the resulting cells, and the obtained mixtures were then reacted at 4°C for 30 minutes. Thereafter, the reaction mixtures were washed twice with FCM buffers, and were then stained with a PE-labeled mouse anti-human IgG antibody (Becton Dickinson) used as a secondary antibody. Flow cytometric analysis was performed using FACSCantoII (Becton Dickinson).

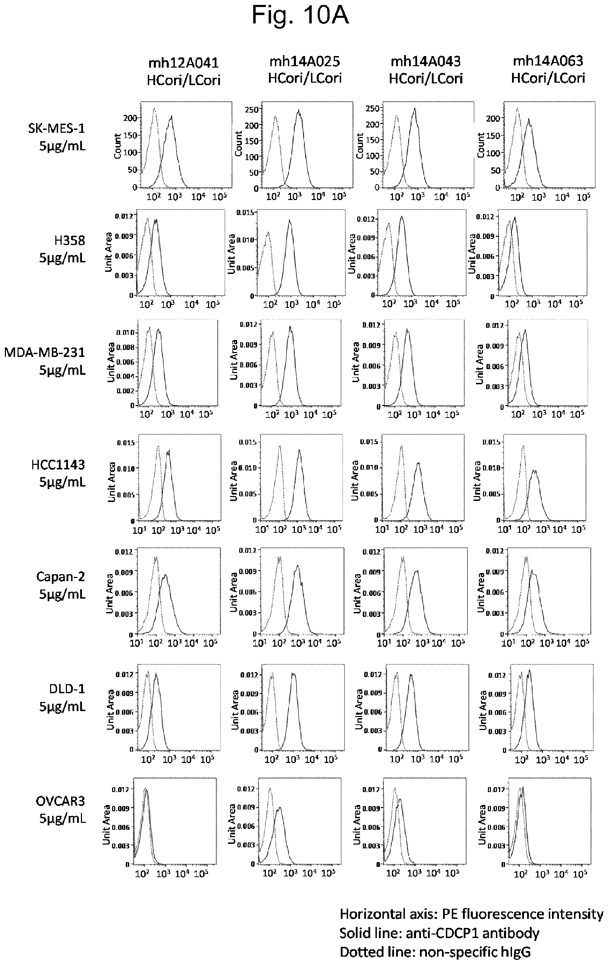

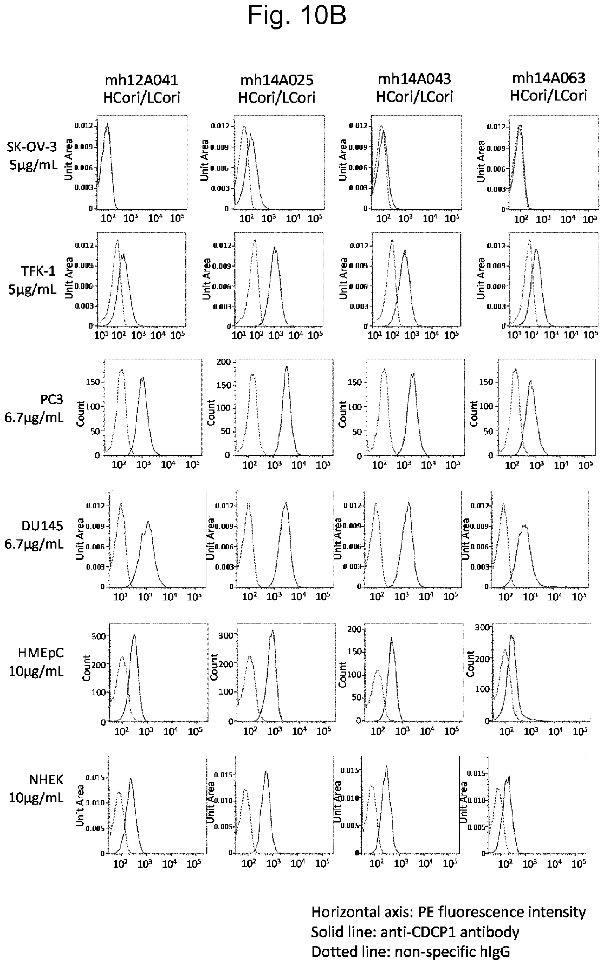

The results are shown in Figure 10A and Figure 10B. The candidate clones all exhibited reactivity to the cancer cells. In addition, the candidate clones also exhibited reactivity to NHEK and HMEpC.

### 11. Reactivity of mouse hybridoma-derived antibodies to bone marrow cells

Healthy human bone marrow-derived mononuclear cells were purchased from any of AllCells, STEMCELL Technologies, and Lonza.

The frozen cell stock was thawed, and was then washed with an IMDM medium (Thermo Fisher Scientific) supplemented with 2% FBS. Thereafter, it was treated with FcR blocking reagent (Miltenyi Biotec K.K.) at room temperature for 15 minutes. A primary antibody sample having a final concentration of 10 µg/mL was added to the reaction mixture as was, and the thus obtained mixture was then reacted at 4°C for 30 minutes. The used primary antibody sample was an antibody sample purified, using protein G sepharose fast flow (GE Healthcare), from the culture supernatant of the hybridoma clone obtained by the screening described in the previous section. The anti-hCDCP1 antibody clone CUB1 (BioLegend) was used as a positive control antibody. A non-specific mouse IgG (monoclonal antibody clone 2E12) manufactured by MBL was used. After washing twice with an FCM buffer, the reaction mixture was reacted with a PE-labeled anti-mouse IgG antibody (Becton Dickinson) at 4°C for 30 minutes. Further, after washing twice with an FCM buffer, the reaction mixture was reacted with an APC-labeled anti-human CD34 antibody (BioLegend) at 4°C for 30 minutes. After washing twice with an FCM buffer, the resultant was suspended in an FCM buffer containing 7-AAD (Becton Dickinson), and flow cytometric observation was then carried out using FACSCantoII (Becton Dickinson).

The reactivity of the evaluated antibodies is shown in Figure 11. Only CD34-APC-positive living cells were gated from the flow cytometry data, and a histogram of the fluorescence values of the PE was then created. As shown in Figure 11, the CUB1 antibody bound more strongly to the CD34-positive cell fraction of the bone marrow mononuclear cells, compared with the non-specific mouse IgG. On the other hand, the reactivity of the following antibody clones listed in Figure 11 was sufficiently low. The reactivity of the antibody clones was equivalent to that of the non-specific mouse IgG even at a comparative antibody concentration of 10 µg/mL, and the reactivity of the antibody clones to the CD34-positive cell fraction was weak to such an extent that it could not be detected.
Clones: 01A2C5, 12A033, 12A038, 12A041, 14A025, and 14C013

### 12. Reactivity of mouse-human chimeric antibodies to bone marrow cells

The same healthy human bone marrow-derived mononuclear cells as those used in the previous section were used. The frozen cell stock was thawed, and was then washed with a FCM buffer. Thereafter, FcR blocking reagent (Miltenyi Biotec K.K.) was added to the resultant, and the obtained mixture was then treated at room temperature for 15 minutes. A primary antibody sample having a final concentration of 10 µg/mL was added to the reaction mixture as was, and the thus obtained mixture was then reacted at 4°C for 30 minutes. The used primary antibody sample was an antibody obtained by purifying the mouse-human chimeric antibody produced in Section 7, using rProtein A Sepharose Fast Flow (GE Healthcare). The anti-hCDCP1 antibody clone CUB1 (BioLegend) was used as a positive control antibody. As an non-CDCP1 specific human IgG (hIgG), an anti-RS virus antibody mentioned in a later section was used. After washing twice with an FCM buffer, the reaction mixture was reacted with a PE-labeled anti-human antibody (SantaCruz), or in the case of the CUB1 antibody, with a PE-labeled anti-mouse IgG antibody (Becton Dickinson) at 4°C for 30 minutes. Further, after washing twice with an FCM buffer, the reaction mixture was reacted with an APC-labeled anti-human CD34 antibody (BioLegend) at 4°C for 30 minutes. After washing twice with an FCM buffer, the resultant was suspended in an FCM buffer containing 7-AAD (Becton Dickinson), and flow cytometric observation was then carried out using FACSCantoII (Becton Dickinson).

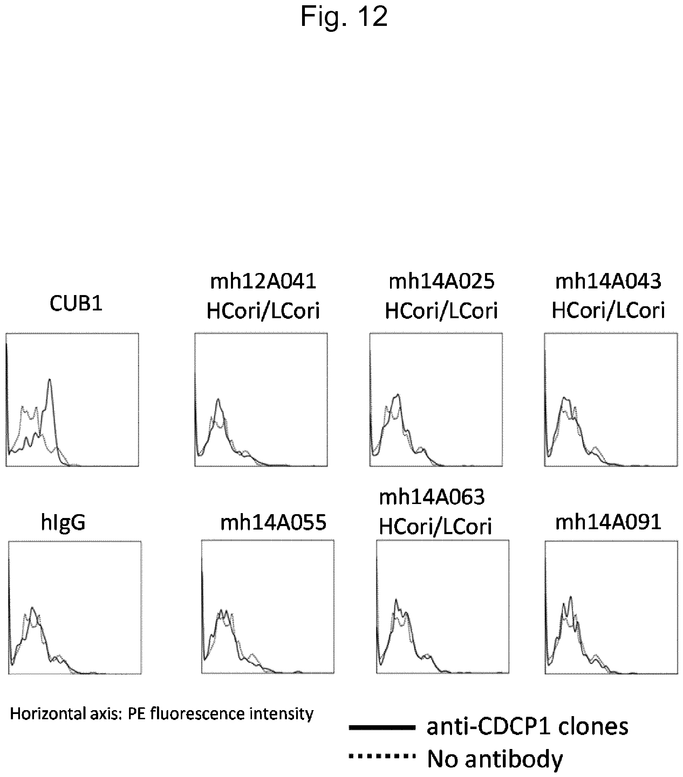

The reactivity of the evaluated antibodies is shown in Figure 12. Only CD34-positive living cells were gated from the flow cytometry data, and a histogram of the fluorescence values of the PE was then created. As shown in Figure 12, the CUB1 antibody bound more strongly to the CD34-positive cell fraction of the bone marrow mononuclear cells, compared with to the cell group without addition of the primary antibody. On the other hand, the reactivity of the following antibody clones listed in Figure 12 was sufficiently low. The antibody clones had staining intensity that was equivalent to that of the cells without addition of the antibody, even when the comparative antibody concentration was set at 10 µg/mL, and the reactivity of the antibody clones to the CD34-positive cell fraction could not be detected.
Clones: mh12A041HCori/LCori, mh14A025HCori/LCori, mh14A043HCori/LCori, mh14A055, mh14A063HCori/LCori, and mh14A091

### 13. Reactivity of biotinylated mouse-human chimeric antibodies to bone marrow cells

Regarding the following antibodies, in order to examine in detail the reactivity thereof to healthy human bone marrow-derived mononuclear cells by a more highly sensitive method, the mouse-human chimeric antibodies were directly biotinylated, and then, using the biotinylated mouse-human chimeric antibodies, flow cytometry was carried out on the bone marrow mononuclear cells.
mh12A041HCori/LCori, mh14A025HCori/LCori, mh14A043HCori/LCori, and mh14A063HCori/LCori

Purified antibodies of the above-described mouse-human chimeric antibodies were biotinylated using the EZ-Link Micro NHS-PEG4-Biotinylation Kit (Thermo Scientific). A 25A11 antibody, a CUB4 antibody, and an anti-RS virus antibody used as comparative controls, were each produced based on the sequences shown below. 25A11 antibody: The heavy chain variable region sequence as set forth in SEQ ID NO: 20 of International Publication WO 2008/133851, and the light chain variable region sequence as set forth in SEQ ID NO: 4 thereof. The heavy chain variable region produced in the present application is shown in SEQ ID NO: 163, and the light chain variable region is shown in SEQ ID NO: 164.
CUB4 antibody: The heavy chain variable region sequence as set forth in SEQ ID NO: 3 of U.S. Patent No. US 8394928, and the light chain variable region sequence as set forth in SEQ ID NO: 15 thereof. The heavy chain variable region produced in the present application is shown in SEQ ID NO: 165, and the light chain variable region is shown in SEQ ID NO: 166.
Anti-RS virus antibody: The sequence registered with PBD database registration number 2hwz. The heavy chain variable region ranges from positions 1 to 120 of 2hwz_H, whereas the light chain variable region ranges from positions 1 to 106 of 2hwz_L. The heavy chain variable region produced in the present application is shown in SEQ ID NO: 167, and the light chain variable region is shown in SEQ ID NO: 168.

Nucleotide sequences encoding these were synthesized according to gene synthesis, and the heavy chain variable region was cloned into a pFUSE-CHIg-hG1 vector, whereas the light chain variable region was cloned into a pFUSE2-CLIg-hk vector. Thereafter, antibody samples purified through the expression by Expi293 cells and the purification with protein A sepharose were used. The anti-RS virus antibody was hereafter used as non-specific human IgG (hIgG).

Likewise, as comparative controls, the purified IgG protein derived from normal human serum (Sigma-Aldrich) and the anti-hCDCP1 antibody clone CUB1 (BioLegend) were also biotinylated by the same method as that described above, and were then subjected to experiments.

The biotinylation valence of each antibody was measured using Biotin Quantification Kit (Thermo Scientific), and it was confirmed that the obtained biotinylation valences were as shown in Table 3 below.

**[Table 3]**

| Antibody | |
|---|---|
| mh12A041HCori/LCori-biotin | 1.37 |
| mh14A025HCori/LCori-biotin | 2.11 |
| mh14A043HCori/LCori-biotin | 2.14 |
| mh14A063HCori/LCori-biotin | 2.67 |
| CUB4-biotin | 2.36 |
| CUB1-biotin | 2.76 |
| 25A11-biotin | 1.96 |
| hIgG-biotin | 1.91 |
| Human serum-derived IgG-biotin | 3.72 |

The same healthy human bone marrow-derived mononuclear cells as those used in the previous section were used. The frozen cell stock was thawed, and was then washed with a FCM buffer, or with an IMDM medium supplemented with 2% FBS. Thereafter, FcR blocking reagent (Miltenyi Biotec K.K.) was added to the resultant, and the obtained mixture was then treated at room temperature for 15 minutes. A primary antibody sample was added to the reaction mixture as was, and the thus obtained mixture was then reacted at 4°C for 30 minutes. After washing twice with an FCM buffer, the reaction mixture was reacted with a mixture consisting of a PE-labeled goat anti-human IgG antibody (Southern Biotechnology), an APC-labeled anti-human CD34 antibody (BioLegend) and a PE-Cy7-labeled anti-human CD45 antibody (BioLegend) at 4°C for 30 minutes. After washing twice with an FCM buffer, the resultant was suspended in an FCM buffer containing 7-AAD (Becton Dickinson), and flow cytometric observation was then carried out using FACSCantoII (Becton Dickinson).

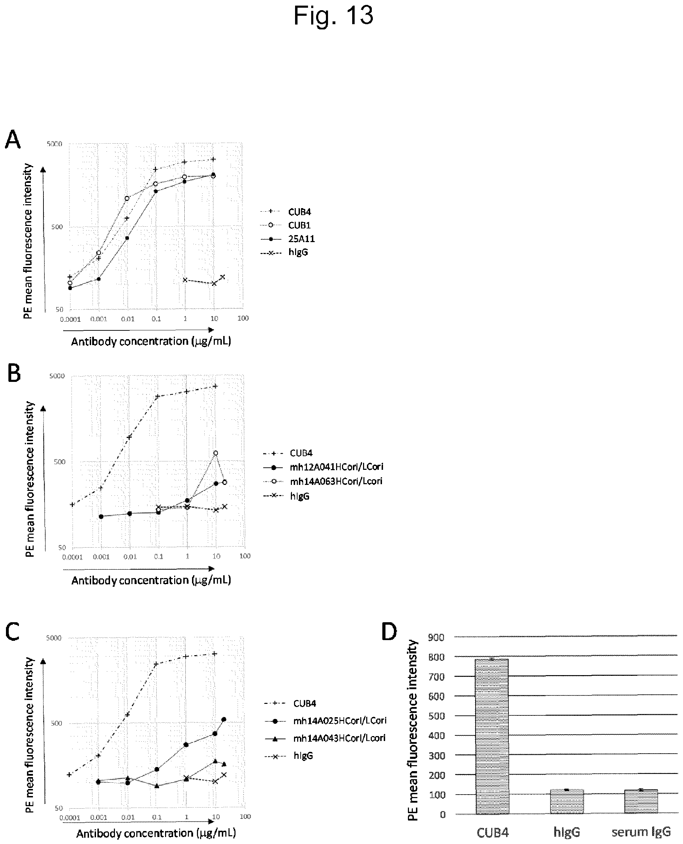

The reactivity of the antibodies to bone marrow cells, which was observed in this experiment, is shown in Figure 13. In this figure, only living cells that were positive to both CD34/CD45 were extracted from the observed results, the average of the PE fluorescence intensity was then calculated, and the relationship with the concentration of the added antibody was then shown. As is found from this figure, the biotinylated antibodies of CUB 1, CUB4, and 25A11 exhibited sufficient reactivity to bone marrow CD34-positive cells even in a low concentration area. In contrast, the reactivity of biotinylated antibodies of the four antibodies mh12A041HCori/LCori, mh14A025HCori/LCori, and mh14A043HCori/LCori to CD34-positive cells was significantly low. In particular, in a comparative antibody concentration of 10 ng/mL, the biotinylated antibodies of CUB 1, CUB4, and 25A11 exhibited strong reactivity, but the biotinylated antibodies of the four antibodies mh12A041HCori/LCori, mh14A025HCori/LCori, and mh14A043HCori/LCori exhibited weak reactivity that was almost the same level as that of a biotinylated non-specific human IgG.

Furthermore, Figure 13D is a view showing a comparison made between the anti-RS virus antibody biotinylated antibody used as a non-specific human IgG in Figures 13A to C and a purified IgG biotinylated protein derived from normal human serum, in terms of the reactivity to bone marrow CD34-positive cells in a comparative antibody concentration of 10 ng/mL. The measurement was carried out on three independent samples, and a mean value of PE mean fluorescence intensity and a standard error are shown in Figure 13D. Thereby, it was demonstrated that the reactivity of the anti-hCDCP1 antibody group described in the present invention was equivalent to that of a human serum-derived IgG protein.

### 14. Humanization of antibodies and reactivity to forced expression cells

Subsequently, the following mouse anti-hCDCP1 antibody sequences were humanized: mh12A041HCv1, mh12A041LCv1, mh14A043HCv2, mh14A043LCv1, mh14A063HCv1, and mh14A063LCori.

Humanization was carried out from the sequence of each antibody variable region according to a CDR transplantation method. The humanized sequence was designed based on the method described in the following study paper: Tsurushita et al., 2005. Design of humanized antibodies: From anti-Tac to Zenapax. Methods 36: 69-83.

First, a three-dimensional molecular model of a mouse antibody was prepared according to a conventional method. Then, based on this molecular model, the residues considered to be important for the structure formation of CDR and the residues considered to be essential for the reaction with the antigen were estimated from the amino acid sequence of the framework region. At the same time, the sequences highly homologous to the heavy chain variable region and light chain variable region of each anti-hCDCP1 antibody were searched from the cDNA sequence database of human antibody heavy chain variable regions and light chain variable regions. Thereafter, a sequence was designed by ligating the sequence of the framework part of the searched human antibody sequence to the CDR sequence of each anti-hCDCP1 antibody, and further, the sequence of residues considered to be essential for the structure formation of CDR and the reaction with the antigen was transplanted into the above-designed sequence, so as to design a humanized antibody sequence. The designed sequences are as shown in the following Table 4. The CDR sequence possessed by the humanized antibody sequence is identical to the sequence of the original mouse antibody, and is the same sequence as the sequence with the above-mentioned sequence number.

**[Table 4]**

| | Variable region sequence | CDR1 | CDR2 | CDR3 |
|---|---|---|---|---|
| h12A041VH1 | SEQ ID NO: 169 | SEQ ID NO: 49 | SEQ ID NO: 50 | SEQ ID NO: 113 |
| h12A041VH2 | SEQ ID NO: 170 | SEQ ID NO: 49 | SEQ ID NO: 50 | SEQ ID NO: 113 |
| h12A041VL | SEQ ID NO: 171 | SEQ ID NO: 117 | SEQ ID NO: 54 | SEQ ID NO: 55 |
| h14A043VH1 | SEQ ID NO: 172 | SEQ ID NO: 73 | SEQ ID NO: 147 | SEQ ID NO: 75 |
| h14A043VH2 | SEQ ID NO: 173 | SEQ ID NO: 73 | SEQ ID NO: 147 | SEQ ID NO: 75 |
| h14A043VL1 | SEQ ID NO: 174 | SEQ ID NO: 149 | SEQ ID NO: 78 | SEQ ID NO: 79 |
| h14A043VL2 | SEQ ID NO: 175 | SEQ ID NO: 149 | SEQ ID NO: 78 | SEQ ID NO: 79 |
| h14A043VL3 | SEQ ID NO: 176 | SEQ ID NO: 149 | SEQ ID NO: 78 | SEQ ID NO: 79 |
| h14A063VH2 | SEQ ID NO: 177 | SEQ ID NO: 89 | SEQ ID NO: 151 | SEQ ID NO: 91 |
| h14A063VH3 | SEQ ID NO: 178 | SEQ ID NO: 89 | SEQ ID NO: 151 | SEQ ID NO: 91 |
| h14A063VH4 | SEQ ID NO: 179 | SEQ ID NO: 89 | SEQ ID NO: 151 | SEQ ID NO: 91 |
| h14A063VL1 | SEQ ID NO: 180 | SEQ ID NO: 93 | SEQ ID NO: 94 | SEQ ID NO: 95 |
| h14A063VL2 | SEQ ID NO: 181 | SEQ ID NO: 93 | SEQ ID NO: 94 | SEQ ID NO: 95 |

It was conceived that, regarding mh12A041HCv1, the serine residue of Kabat No. 32 was mutated to a phenylalanine residue, and regarding mh12A041LCv1, the histidine residue of Kabat No. 27d was mutated to a tyrosine residue, or the serine residue of Kabat No. 27a and the asparagine residue of Kabat No. 28 were simultaneously mutated to an asparagine residue and an aspartic acid residue, respectively, so that the affinity to an antigen seemed to be improved Thus, we produced the sequences shown in the following Table 5, which comprised the aforementioned mutations.

**[Table 5]**

| | Variable region sequence | CDRH1 | CDRH2 | CDRH3 |
|---|---|---|---|---|
| h12A041VH1A | SEQ ID NO: 182 | SEQ ID NO: 183 | SEQ ID NO: 50 | SEQ ID NO: 113 |
| h12A041VH2A | SEQ ID NO: 184 | SEQ ID NO: 185 | SEQ ID NO: 50 | SEQ ID NO: 113 |
| h12A041VLA | SEQ ID NO: 185 | SEQ ID NO: 186 | SEQ ID NO: 54 | SEQ ID NO: 55 |
| h12A041VLB | SEQ ID NO: 187 | SEQ ID NO: 188 | SEQ ID NO: 54 | SEQ ID NO: 55 |

The DNA sequence encoding the amino acid sequence of the designed variable region was synthesized according to gene synthesis. The heavy chain variable region sequence DNA was connected with a human antibody secretion signal peptide or a human IL-2 secretion signal peptide, and was then cloned into pFUSE-CHIg-hg1 as a vector comprising a human IgG1 constant region. The light chain variable region sequence DNA was connected with a human antibody secretion signal peptide or a human IL-2 secretion signal peptide, and was then cloned into a pFUSE2-CLIg-hk vector as a vector comprising a human Igκ constant region.

The cloned plasmid was introduced into Expi293 cells, using Expifectamine, and the antibody was allowed to express in the culture solution. Heavy chain and light chain vectors were combined in the following patterns and were then expressed: h12A041: VH1/VL, VH2/VL, VH1/VLA, VH2/VLA, VH1/VLB, VH2/VLB, VH1A/VL, VH2A/VL, VH1A/VLA, VH2A/VLA, VH1A/VLB, and VH2A/VLB h14A043: VH1/VL1, VH1/VL2, VH1/VL3, VH2/VL1, VH2/VL2, and VH2/VL3 h14A063: VH2/VL1, VH2/VL2, VH3/VL1, VH3/VL2, VH4/VL1, and VH4/VL2.

The culture solution was recovered and was then cleaned through a filter, and the concentration of the antibody in the culture solution was then measured according to an AlphaLISA method.

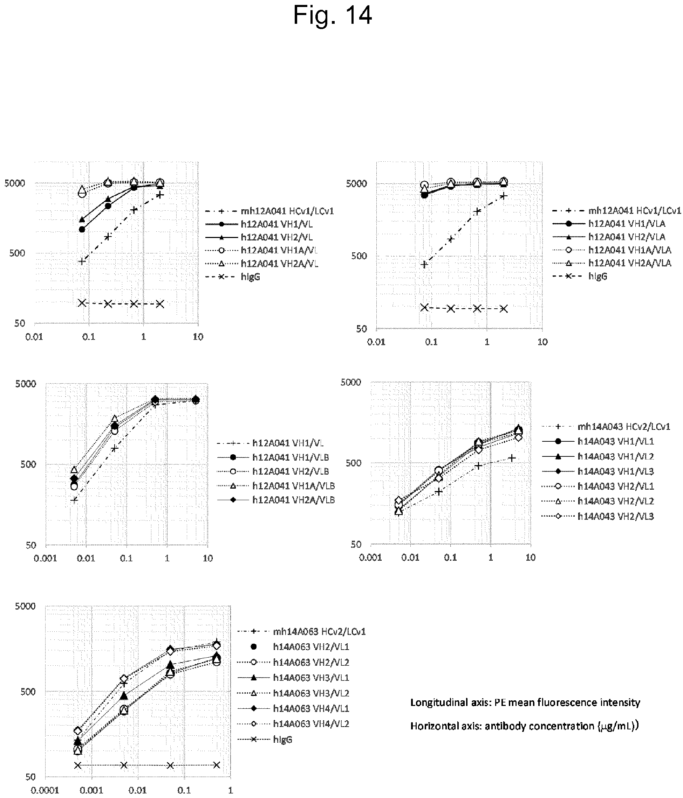

Using the produced humanized anti-hCDCP1 antibody protein and the original mouse-human chimeric antibody clone used as a comparative control, the reactivity of these antibodies to hCDCP1 was examined according to flow cytometry. Ba/F3-hCDCP1 cells were used as target cells. As a secondary antibody, a PE-labeled mouse anti-human IgG antibody (Becton Dickinson) was used. As is apparent from the results of this experiment shown in Figure 14, the designed humanized antibodies all exhibited reactivity that was equivalent to or greater than the reactivity of the original mouse-human chimeric antibody. These results showed that the produced humanized anti-hCDCP1 antibody group sufficiently retains reactivity to hCDCP1.

### 15. Reactivity of humanized antibodies to bone marrow cells

Purified proteins of antibodies having the following combinations of heavy chains and light chains were directly biotinylated according to the same method as that described in the previous section:
h12A041: VH1/VL, VH1/VLA, VH1/VLB, and VH1A/VL
h14A043: VH1/VL1, VHI/VL2, VH1/VL3, VH2/VL1, VH2/VL2, and VH2/VL3
h14A063: VH4/VL1 and VH4/VL2.

As antibodies used as comparative controls, the 25A11 antibody, the CUB4 antibody and the CUB1 antibody produced in Section 13 were used, and an anti-RS virus antibody was used as a non-specific human IgG antibody. In addition, normal human serum-derived purified IgG proteins were used. These antibodies were directly biotinylated. The biotinylation valence of each antibody was measured using Biotin Quantification Kit (Thermo Scientific), and the measurement results are as shown in the following Table 6.

**[Table 6]**

| Antibody | |
|---|---|
| h12A041VH1/VL-biotin | 1.61 |
| h12A041VH1/VLA-biotin | 2.02 |
| h12A041VH1/VLB-biotin | 1.51 |
| h12A041VH1A/VL-biotin | 2.37 |
| h14A043VH1/VL1-biotin | 2.14 |
| h14A043VH1/VL2-biotin | 1.89 |
| h14A043VH1/VL3-biotin | 1.94 |
| h14A043VH2/VL1-biotin | 1.89 |
| h14A043VH2/VL2-biotin | 2.30 |
| h14A043VH2/VL3-biotin | 2.14 |
| h14A063VH4/VL1-biotin | 1.74 |
| h14A063VH4/VL2-biotin | 1.35 |

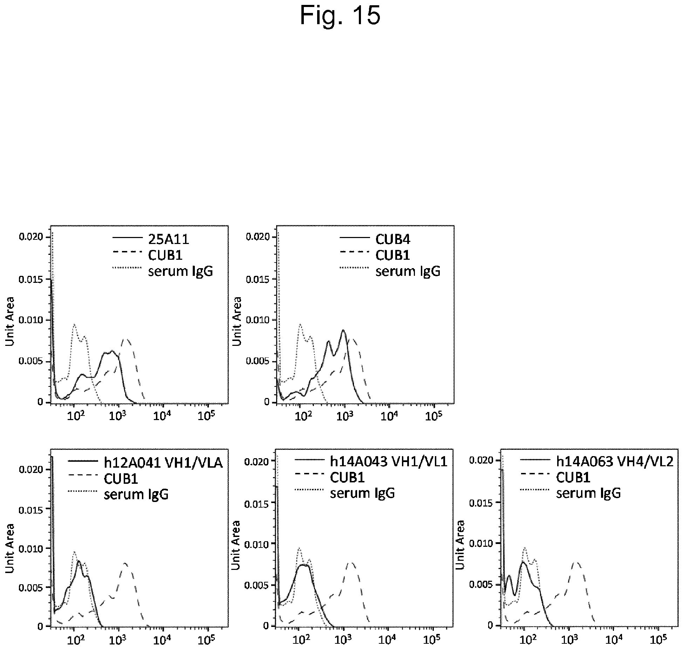

Using these antibodies, the reactivity of the antibodies to healthy human bone marrow-derived mononuclear cells was observed by flow cytometry according to the same method as that described in the previous section. Figure 15 includes histograms showing the results obtained by binding the following biotinylated antibodies to healthy human bone marrow-derived mononuclear cells at a concentration of 10 ng/mL, and then observing the obtained mixtures by flow cytometry, so as to observe the PE fluorescence intensity of a living cell population positive to both CD34 and CD45: 25A11-biotin, CUB4-biotin, CUB1-biotin, h12A041VH1/VLA-biotin, h14A043VH1/VL1-biotin, h14A063VH4/VL2-biotin, and human serum IgG-biotin.

From these results, it was demonstrated that the antibody of the present invention had extremely low reactivity to CD34-positive cells in the bone marrow, compared with the antibody as a comparative control, and had almost no reactivity, as with IgG in human serum.

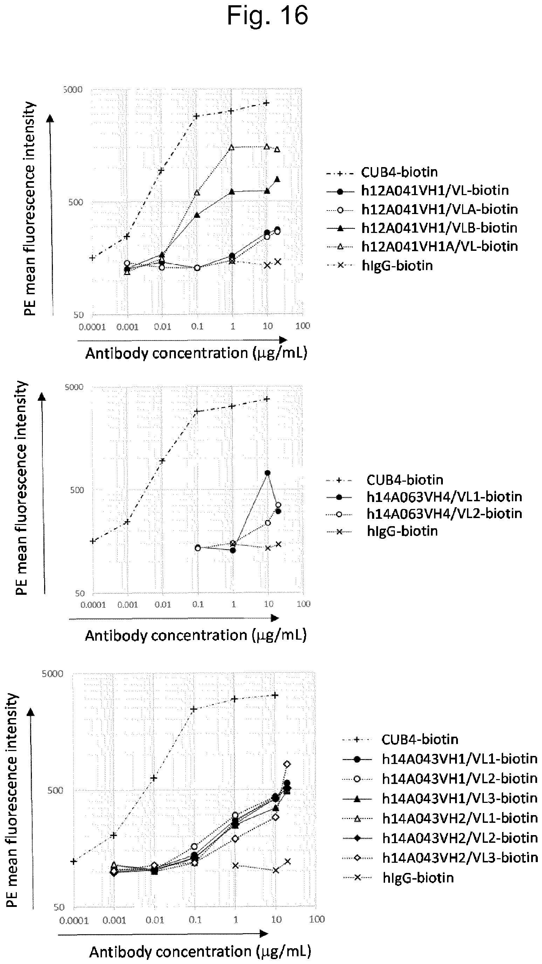

Further, regarding the antibody group shown in Table 6, the same experiment was carried out with the exception that the concentration of an antibody to act on the cells was changed. Figure 16 shows the measurement results of flow cytometry, from which the concentration dependence of the mean fluorescence intensity of PE in living CD34-positive cells was shown in the form of a log-log graph. As a result, the CUB4 antibody exhibited significant binding property to CD34-positive cells in the bone marrow, whereas the antibodies shown in Table 6 all exhibited sufficiently low reactivity to the antibody as a positive control, and exhibited the same level of reactivity as the non-specific human IgG antibody observed as a negative control, at least, in a comparative antibody concentration of 10 ng/mL. From these results, it was considered that the antibody of the present invention would have low reactivity to hematopoietic stem cells, and thus that modified antibodies having antitumor activity derived from the present antibody would also have low cytotoxicity against bone marrow cells.

### 16. Production of PBD-conjugated antibodies

With regard to the mouse-human chimeric antibodies prepared in Section 7 and the humanized antibodies prepared in Section 14, as shown below, and the aforementioned anti-RS virus antibody used as non-specific human IgG, PBD (Pyrrolobenzodiazepine) used as an anticancer agent was directly bound to each of the purified antibodies thereof, so as to produce antibody-drug conjugates. The cytotoxicity thereof was evaluated.
mh12A041HCori/LCori, mh14A025HCori/LCori, mh14A043HCori/LCori, and mh14A063HCori/LCori
h12A041VH1/VLA, h14A043VH1/VL1, and h14A063VH4/VL2

TCEP (Tris(2-carboxyethyl)phosphine Hydrochloride) was added to 2 mg of the purified antibody to cleave the disulfide bond, and thereafter, maleimide-PEG4-Val-Ala-PBD was bound to the antibody according to an addition-binding reaction between maleimide and cysteine. Thereafter, unreacted maleimide-PEG4-Val-Ala-PBD was removed by using a Sephadex G50 column, and the buffer was replaced with PBS (pH 7.2). The names of the prepared PBD-conjugated antibodies are as follows. The names of the PBD-conjugated antibodies are shown together with the number of bound PBD molecules (DAR) per antibody molecule, which was measured based on the ratio of the absorption at 333 nm to the absorption at 280 nm.
Mouse-human chimeric antibody-PBD conjugated antibodies: mh12A041-PBD (DAR 2.1); mh14A025-PBD (DAR 1.7); mh14A043-PBD (DAR 1.8); and mh14A063-PBD (DAR 2.0)
Humanized antibody-PBD conjugated antibodies: h12A041-PBD (DAR 3.8); h14A043-PBD (DAR 3.6); and h14A063-PBD (DAR 3.4)
Anti-RS virus antibody-PBD conjugated antibody: hIgG-PBD (DAR 2.2)

### 17. Cytotoxicity of mouse-human chimeric antibody-PBD conjugated antibodies against cancer cells

The cytotoxicity of the mouse-human chimeric antibody-PBD conjugated antibodies prepared in the previous section against cultured cells was evaluated by the following method. The cancer cell lines and the primary cultured cells derived from normal tissues, which were described in the previous section, were used herein.

The cells were cultured in a plastic dish using a suitable medium. The cells were exfoliated by a trypsin treatment, and 1000 cells or 2000 cells per well were seeded in a 96-well flat bottom plate. The cells were cultured at 37°C until the next day, and a PBD-conjugated antibody diluted to each predetermined concentration with a medium was then added to the cultured cells, and thereafter, the obtained mixture was incubated at 37°C in an incubator for 3 days (DU145), for 5 days (PC3), or for 7 days (other cells). Thereafter, the viability of the cells was measured using Cell Titer Glo (Promega). Two wells were treated under the same conditions with respect to the concentration of each PBD-conjugated antibody, and the mean value of the two wells was adopted as a measured value.

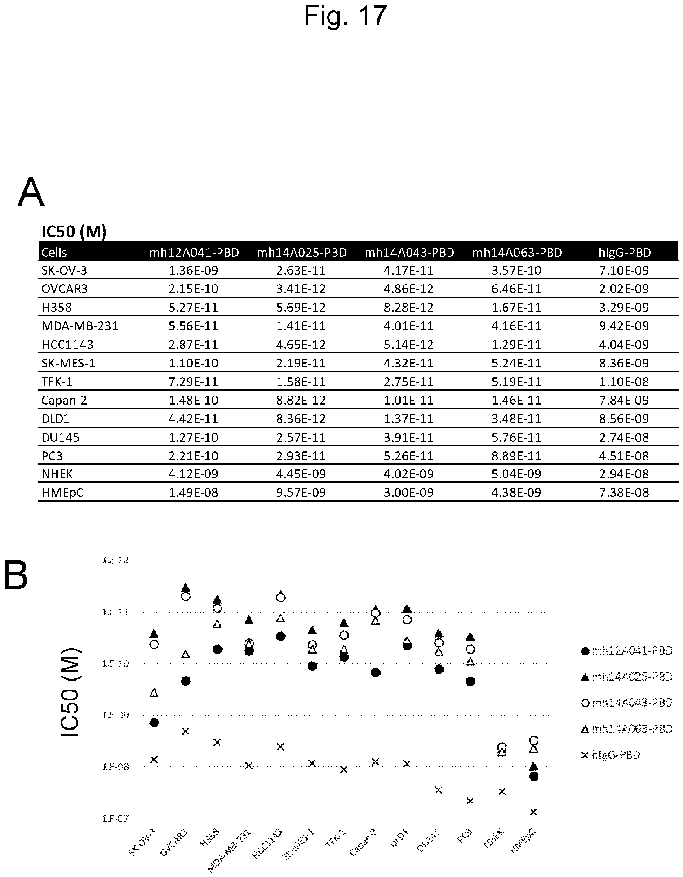

The tested four anti-hCDCP1 mouse-human chimeric antibody-PBD conjugated antibodies exhibited strong cytotoxicity against the cell lines of lung cancer, breast cancer, ovarian cancer, prostate cancer, colorectal cancer, pancreatic cancer, and bile duct cancer. The tested anti-hCDCP1 mouse-human chimeric antibody-PBD conjugated antibodies also exhibited cytotoxicity against mammary epithelial cells (HMEpC) and normal human epidermal keratinocytes (NHEK) at high concentrations, but the cytotoxic level thereof was weak, compared with the cytotoxicity against the aforementioned cancer cell lines. The 50% inhibition concentrations (IC50) calculated from the experimental results are shown in Figure 17A, and a graph formed from the IC50 is shown in Figure 17B. As can be seen from these figures, the cytotoxicity of each antibody-PBD conjugate against NHEK and HMEpC was weaker than the cytotoxicity against the cancer cell lines.

### 18. Cytotoxicity of humanized antibody-PBD conjugated antibodies against cancer cells

The cytotoxicity of the humanized antibody-PBD conjugated antibodies prepared in Section 16 against cultured cells was evaluated by the same method as that described in Section 17. The cell lines PC3, DU145, HCT116, MDA-MB-231, H358 and SK-OV-3, and normal human epidermal keratinocytes (NHEK) were used, and these cell lines were the same as those used in Section 10.

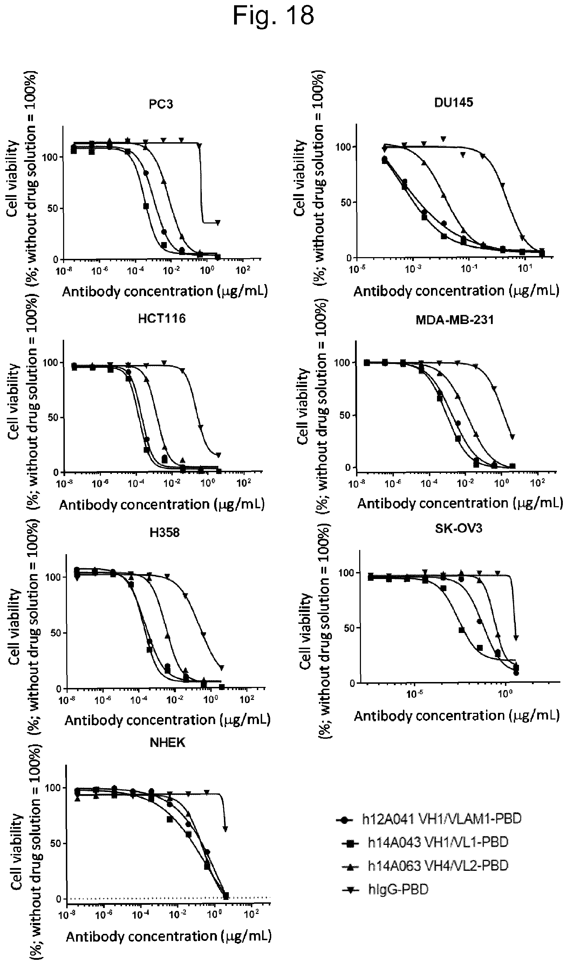

The cells were exfoliated by a trypsin treatment, and 1000 cells or 2000 cells per well were seeded in a 96-well flat bottom plate. The cells were cultured at 37°C until the next day, and a PBD-conjugated antibody diluted to each predetermined concentration with a medium was then added to the cultured cells, and thereafter, the obtained mixture was incubated at 37°C in an incubator for 3 days (DU145), for 5 days (PC3), or for 7 days (other cells). Thereafter, the viability of the cells was measured using Cell Titer Glo (Promega). Two wells were treated under the same conditions with respect to the concentration of each PBD-conjugated antibody, and the mean value of the two wells was adopted as a measured value. The results are shown in Figure 18.

The tested three humanized anti-hCDCP1 antibody PBD conjugates exhibited strong cytotoxicity against the cell lines PC3, DU145, HCT116, MDA-MB-231, and H358. The cytotoxicity of these humanized anti-hCDCP1 antibody PBD conjugates against SK-OV-3 cells was weaker than the cytotoxicity thereof against the cell lines PC3, DU145, HCT116, MDA-MB-231, and H358. The humanized anti-hCDCP1 antibody PBD conjugates also exhibited cytotoxicity against normal human epidermal keratinocytes (NHEK) at a high concentration, but the cytotoxic level thereof was weak, compared with the cytotoxicity against the aforementioned cancer cell lines. The 50% inhibition concentrations (IC50) calculated from the experimental results are as shown in the following table.

**[Table 7]**

| IC50 (*µ*g/mL) | h12A041VH1/VLA-PBD | h14A048VH1/VL1-PBD | h14A068VH4/VL2-PBD |
|---|---|---|---|
| PC3 | 1.19×10⁻³ | 3.57 × 10⁻⁴ | 7.90×10⁻³ |
| DU145 | 2.55 × 10⁻⁴ | 5.02 × 10⁻⁴ | 1.44 × 10⁻² |
| HCT116 | 2.18 × 10⁻⁴ | 1.55 × 10⁻⁴ | 1.51 × 10⁻³ |
| MDA-MB-231 | 2.01 × 10⁻³ | 9.96 × 10⁻⁴ | 1.32 × 10⁻² |
| H358 | 2.43 × 10⁻⁴ | 2.04 × 10⁻⁴ | 3.32 × 10⁻³ |
| SK-OV3 | 6.11 × 10⁻² | 2.70 × 10⁻³ | 2.66 × 10⁻¹ |
| NHEK | 2.39 | 3.46 × 10⁻¹ | 2.62 × 10⁻¹ |

As is found from Table 7, the cytotoxicity of each antibody-PBD conjugate against NHEK was weak, compared with the cytotoxicity thereof to the cancer cell lines.

### 19. Antitumor activity of mouse-human chimeric antibody-PBD conjugated antibodies against xenograft models

Subsequently, the *in vivo* antitumor activity of anti-CDCP1 mouse-human chimeric antibody-PBD conjugated antibodies was evaluated by the following method. As mice, scid mice (C.B17/Icr-scidJcl; CLEA Japan Inc.) or nude mice (BALB/cAJclnu/nu; CLEA Japan Inc.), both of which were 6- to 7-week-old female mice, were used. PC3 cells were purchased from ATCC. The used PC3 cells were cultured in a Ham's F-12K medium (Wako Pure Chemical Industries, Ltd.) supplemented with 7% FBS and 10 µg/mL Gentamicin (Thermo Scientific). The cells were exfoliated with 0.25% trypsin/0.02% EDTA (Thermo Scientific), and were then washed with PBS. Thereafter, the resulting cells (5 × 10⁶ cells per mouse) were mixed with Matrigel growth factor reduced phenol red-free (Corning) at a ratio of 1 : 1, and the obtained mixture was then subcutaneously transplanted into the right flank of the mice.

When the tumors grew and the average tumor volume exceeded 100 mm³, the mice were randomized and grouped (8 mice per group) based on the tumor volume of each mouse. On the same day or the next day of the grouping, a test drug was administered once at a dose of 10 µL/g per mouse body weight through the caudal vein.

The measurement of the tumor size and the body weight was started 3 or 4 days after the cell transplantation, and the measurement was then performed at a frequency of twice a week. Using a caliper, both the minor axis and the major axis of the tumor were measured. The tumor size was calculated according to the expression: (minor axis mm)² × (major axis mm) × 3.14/6.

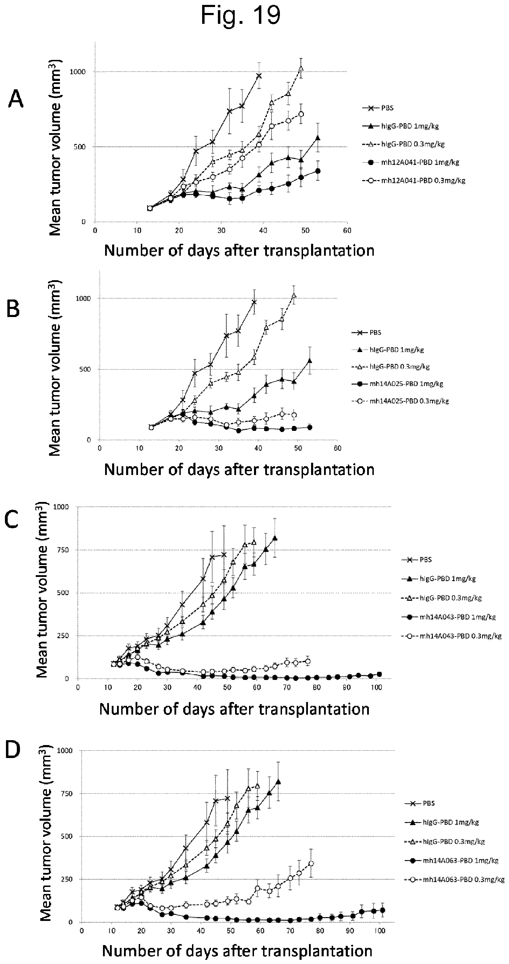

The experimental results using the scid mouse models are shown in Figures 19A and B. Both mh12A041-PBD and mh14A025-PBD significantly suppressed tumors in both the 1 mg/kg and 0.3 mg/kg groups, compared with a group that was administered with the same concentration of non-specific hIgG-PBD.

The experimental results using the nude mouse models are shown in Figures 19C and D. Both mh14A043-PBD and mh14A063-PBD significantly suppressed tumors in both the 1 mg/kg and 0.3 mg/kg groups, compared with a group that was administered with the same concentration of non-specific hIgG-PBD.

### 20. Antitumor activity of humanized antibody-PBD conjugated antibodies against xenograft models

Subsequently, the *in vivo* antitumor activity of anti-CDCP1 humanized antibody-PBD conjugated antibodies was evaluated by the following method. As mice, nude mice (BALB/cAJcl-nu/nu; CLEA Japan Inc.), which were 6- to 7-week-old female mice, were used. PC3 cells and HCT116 cells were purchased from ATCC. The used PC3 cells were cultured in a Ham's F-12K medium (Wako Pure Chemical Industries, Ltd.) supplemented with 7% FBS and 10 µg/mL Gentamicin (Thermo Scientific). The used HCT116 cells were cultured in a McCoy's 5A medium (Thermo Scientific) supplemented with 10% FBS and 1% penicillin/streptomycin (Nacalai Tesque, Inc.).

The cells were exfoliated with 0.25% trypsin/0.02% EDTA (Thermo Scientific), and were then washed with PBS. Thereafter, the resulting cells (5 × 10⁶ cells per mouse) were mixed with Matrigel growth factor reduced phenol red-free (Corning) at a ratio of 1 : 1, and the obtained mixture was then subcutaneously transplanted into the right flank of the mice.

When the tumors grew and the average tumor volume exceeded 100 mm³, the mice were randomized and grouped (8 mice per group) based on the tumor volume of each mouse. On the same day of the grouping, a test drug was administered once at a dose of 10 µL/g per mouse body weight through the caudal vein.

The tumor size and the body weight were measured by the same method as that described in Section 19.

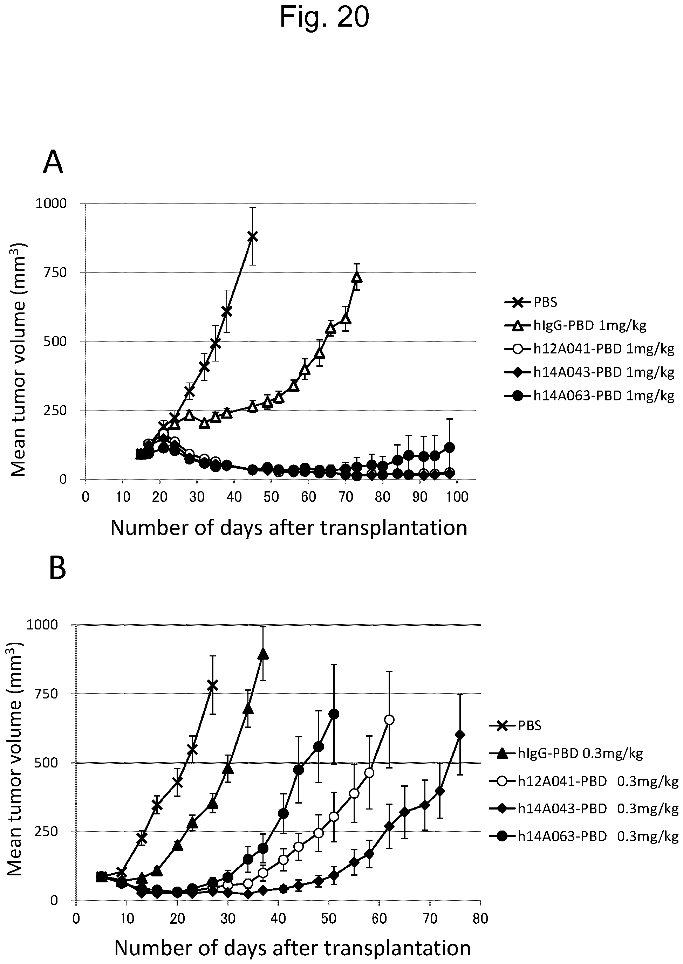

The experimental results using the xenograft models with the PC3 cells are shown in Figure 20A. All of h12A041-PBD, h14A043-PBD, and h14A063-PBD significantly suppressed tumors by a single administration of 1 mg/kg, compared with a group that was administered with the same concentration of non-specific hIgG-PBD.

The experimental results using the xenograft models with the HCT116 cells are shown in Figure 20B. All of h12A041-PBD, h14A043-PBD, h14A063-PBD significantly suppressed tumors by a single administration of 0.3 mg/kg, compared with a group that was administered with the same concentration of non-specific hIgG-PBD.

### 21. Production of MMAE-conjugated antibodies

Regarding the following humanized antibodies prepared in Section 14, an antibody-drug conjugate, in which MMAE (monomethyl auristatin E) used as an anticancer agent was directly bound to a purified antibody of each humanized antibody, was prepared, and the cytotoxicity thereof was then evaluated:
h14A043VH1/VL1 and h14A063VH4/VL2.

TCEP (Tris(2-carboxyethyl)phosphine Hydrochloride) was added to approximately 30 mg of the purified antibody to cleave a disulfide bond thereof, and maleimidocaproyl-valine-citruline-p-aminobenzyloxycarbonyl-monomethyl auristatin E (MC-vc-PAB-MMAE) was then bound to the antibody according to an addition-binding reaction. Thereafter, unreacted MC-vc-PAB-MMAE was removed by using a Sephadex G50 column, and the buffer was then replaced with PBS (pH 7.2). The number of bound MMAE molecules per antibody molecule was measured based on the ratio of the absorption at 248 nm to the absorption at 280 nm. The number of bound MMAE molecules to h14A043VH1/VL1 was measured to be approximately 3.9, whereas the number of bound MMAE molecules to h14A063VH4/VL2 was measured to be approximately 4.0. The names of the prepared MMAE-conjugated antibodies are as follows:
h14A043-MMAE; and h14A063-MMAE.

### 22. Antitumor activity of humanized antibody MMAE-conjugated antibodies against xenograft models

Subsequently, the *in vivo* antitumor activity of humanized antibody MMAE-conjugated antibodies was evaluated by the following method. As mice, nude mice (BALB/cAJcl-nu/nu; CLEA Japan Inc.), which were 6- to 7-week-old female mice, were used. HCT116 cells were purchased from ATCC. The used HCT116 cells were cultured by the same method as that described in Section 20.

The cells were exfoliated with 0.25% trypsin/0.02% EDTA (Thermo Scientific), and were then washed with PBS. Thereafter, the resulting cells (5 × 10⁶ cells per mouse) were mixed with Matrigel growth factor reduced phenol red-free (Corning) at a ratio of 1 : 1, and the obtained mixture was then subcutaneously transplanted into the right flank of the mice.

When the tumors grew and the average tumor volume exceeded 100 mm³, the mice were randomized and grouped (8 mice per group) based on the tumor volume of each mouse. Setting the grouping day as an initial administration day, a test drug and a phosphate buffered saline (PBS) as a solvent that was used as a negative control were administered twice a week, 4 times in total. Administration was carried out at a dose of 10 µL/g per mouse body weight through the caudal vein. The tumor size and the body weight were measured by the same method as that described in Section 19.

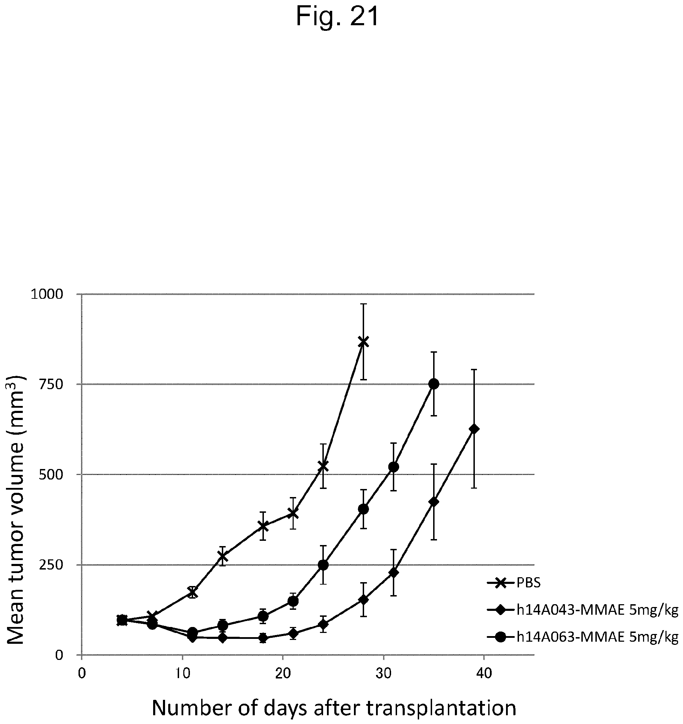

The experimental results are shown in Figure 21. Both h14A043-PBD and h14A063-PBD significantly suppressed tumors by 4 times administration at a single dose of 5 mg/kg, compared with a PBS administration group.

### Industrial Applicability

The antibody provided by the present invention and an antigen-binding fragment thereof are considered to play an important role in provision of the prevention or treatment of cancer, the development of a preventive or therapeutic agent for cancer, etc. Accordingly, it is expected that the present invention will be utilized in the

## Claims

1. An antibody that binds to human CDCP1 (CUB domain-containing protein 1), which has low binding property to human CD34-positive cells, or an antigen-binding fragment thereof.

2. The antibody according to claim 1, wherein the concentration of a comparative antibody in the evaluation of the binding property to human CD34-positive cells is any concentration of 10 ng/ml or more, or the antigen-binding fragment thereof.

3. An antibody that the amino acid sequences of CDRs (complementarity determining regions) 1 to 3 thereof satisfy any of the following (A) to (K), or an antigen-binding fragment thereof:
(A) an antibody having:
heavy chain CDR1 comprising the amino acid sequence as set forth in SEQ ID NO: 25,
heavy chain CDR2 comprising the amino acid sequence as set forth in SEQ ID NO: 26,
heavy chain CDR3 comprising the amino acid sequence as set forth in SEQ ID NO: 27,
light chain CDR1 comprising the amino acid sequence as set forth in SEQ ID NO: 29,
light chain CDR2 comprising the amino acid sequence as set forth in SEQ ID NO: 30, and
light chain CDR3 comprising the amino acid sequence as set forth in SEQ ID NO: 31;
(B) an antibody having:
heavy chain CDR1 comprising the amino acid sequence as set forth in SEQ ID NO: 33,
heavy chain CDR2 comprising the amino acid sequence as set forth in SEQ ID NO: 34,
heavy chain CDR3 comprising the amino acid sequence as set forth in SEQ ID NO: 35,
light chain CDR1 comprising the amino acid sequence as set forth in SEQ ID NO: 37,
light chain CDR2 comprising the amino acid sequence as set forth in SEQ ID NO: 38, and
light chain CDR3 comprising the amino acid sequence as set forth in SEQ ID NO: 39;
(C) an antibody having:
heavy chain CDR1 comprising the amino acid sequence as set forth in SEQ ID NO: 41,
heavy chain CDR2 comprising the amino acid sequence as set forth in SEQ ID NO: 42,
heavy chain CDR3 comprising the amino acid sequence as set forth in SEQ ID NO: 43,
light chain CDR1 comprising the amino acid sequence as set forth in SEQ ID NO: 45,
light chain CDR2 comprising the amino acid sequence as set forth in SEQ ID NO: 46, and
light chain CDR3 comprising the amino acid sequence as set forth in SEQ ID NO: 47;
(D) an antibody having:
heavy chain CDR1 comprising the amino acid sequence as set forth in SEQ ID NO: 49,
heavy chain CDR2 comprising the amino acid sequence as set forth in SEQ ID NO: 50,
heavy chain CDR3 comprising the amino acid sequence as set forth in SEQ ID NO: 51,
light chain CDR1 comprising the amino acid sequence as set forth in SEQ ID NO: 53,
light chain CDR2 comprising the amino acid sequence as set forth in SEQ ID NO: 54, and
light chain CDR3 comprising the amino acid sequence as set forth in SEQ ID NO: 55;
(E) an antibody having:
heavy chain CDR1 comprising the amino acid sequence as set forth in SEQ ID NO: 57,
heavy chain CDR2 comprising the amino acid sequence as set forth in SEQ ID NO: 58,
heavy chain CDR3 comprising the amino acid sequence as set forth in SEQ ID NO: 59,
light chain CDR1 comprising the amino acid sequence as set forth in SEQ ID NO: 61,
light chain CDR2 comprising the amino acid sequence as set forth in SEQ ID NO: 62, and
light chain CDR3 comprising the amino acid sequence as set forth in SEQ ID NO: 63;
(F) an antibody having:
heavy chain CDR1 comprising the amino acid sequence as set forth in SEQ ID NO: 65,
heavy chain CDR2 comprising the amino acid sequence as set forth in SEQ ID NO: 66,
heavy chain CDR3 comprising the amino acid sequence as set forth in SEQ ID NO: 67,
light chain CDR1 comprising the amino acid sequence as set forth in SEQ ID NO: 69,
light chain CDR2 comprising the amino acid sequence as set forth in SEQ ID NO: 70, and
light chain CDR3 comprising the amino acid sequence as set forth in SEQ ID NO: 71;
(G) an antibody having:
heavy chain CDR1 comprising the amino acid sequence as set forth in SEQ ID NO: 73,
heavy chain CDR2 comprising the amino acid sequence as set forth in SEQ ID NO: 74,
heavy chain CDR3 comprising the amino acid sequence as set forth in SEQ ID NO: 75,
light chain CDR1 comprising the amino acid sequence as set forth in SEQ ID NO: 77,
light chain CDR2 comprising the amino acid sequence as set forth in SEQ ID NO: 78, and
light chain CDR3 comprising the amino acid sequence as set forth in SEQ ID NO: 79;
(H) an antibody having:
heavy chain CDR1 comprising the amino acid sequence as set forth in SEQ ID NO: 81,
heavy chain CDR2 comprising the amino acid sequence as set forth in SEQ ID NO: 82,
heavy chain CDR3 comprising the amino acid sequence as set forth in SEQ ID NO: 83,
light chain CDR1 comprising the amino acid sequence as set forth in SEQ ID NO: 85,
light chain CDR2 comprising the amino acid sequence as set forth in SEQ ID NO: 86, and
light chain CDR3 comprising the amino acid sequence as set forth in SEQ ID NO: 87;
(I) an antibody having:
heavy chain CDR1 comprising the amino acid sequence as set forth in SEQ ID NO: 89,
heavy chain CDR2 comprising the amino acid sequence as set forth in SEQ ID NO: 90,
heavy chain CDR3 comprising the amino acid sequence as set forth in SEQ ID NO: 91,
light chain CDR1 comprising the amino acid sequence as set forth in SEQ ID NO: 93,
light chain CDR2 comprising the amino acid sequence as set forth in SEQ ID NO: 94, and
light chain CDR3 comprising the amino acid sequence as set forth in SEQ ID NO: 95;
(J) an antibody having:
heavy chain CDR1 comprising the amino acid sequence as set forth in SEQ ID NO: 97,
heavy chain CDR2 comprising the amino acid sequence as set forth in SEQ ID NO: 98,
heavy chain CDR3 comprising the amino acid sequence as set forth in SEQ ID NO: 99,
light chain CDR1 comprising the amino acid sequence as set forth in SEQ ID NO: 101,
light chain CDR2 comprising the amino acid sequence as set forth in SEQ ID NO: 102, and
light chain CDR3 comprising the amino acid sequence as set forth in SEQ ID NO: 103; and
(K) an antibody having:
heavy chain CDR1 comprising the amino acid sequence as set forth in SEQ ID NO: 105,
heavy chain CDR2 comprising the amino acid sequence as set forth in SEQ ID NO: 106,
heavy chain CDR3 comprising the amino acid sequence as set forth in SEQ ID NO: 107,
light chain CDR1 comprising the amino acid sequence as set forth in SEQ ID NO: 109,
light chain CDR2 comprising the amino acid sequence as set forth in SEQ ID NO: 110, and
light chain CDR3 comprising the amino acid sequence as set forth in SEQ ID NO: 111.

4. An antibody that the amino acid sequences of CDRs (complementarity determining regions) 1 to 3 thereof satisfy either the following (L) or (M), or an antigen-binding fragment thereof:
(L) an antibody having:
heavy chain CDR1 comprising the amino acid sequence as set forth in SEQ ID NO: 49,
heavy chain CDR2 comprising the amino acid sequence as set forth in SEQ ID NO: 50,
heavy chain CDR3 comprising the amino acid sequence as set forth in SEQ ID NO: 51,
light chain CDR1 comprising the amino acid sequence as set forth in SEQ ID NO: 53,
light chain CDR2 comprising the amino acid sequence as set forth in SEQ ID NO: 54, and
light chain CDR3 comprising the amino acid sequence as set forth in SEQ ID NO: 55; or
(M) the antibody satisfying the above (L), which has heavy chain and light chain variable regions, in which the amino acid at position 102 of the heavy chain and the amino acid at positions 28 and 29 of the light chain according to Kabat definition are substituted with other amino acids.

5. An antibody that the amino acid sequences of CDRs (complementarity determining regions) 1 to 3 thereof satisfy either the following (N) or (O), or an antigen-binding fragment thereof:
(N) an antibody having:
heavy chain CDR1 comprising the amino acid sequence as set forth in SEQ ID NO: 65,
heavy chain CDR2 comprising the amino acid sequence as set forth in SEQ ID NO: 66,
heavy chain CDR3 comprising the amino acid sequence as set forth in SEQ ID NO: 67,
light chain CDR1 comprising the amino acid sequence as set forth in SEQ ID NO: 69,
light chain CDR2 comprising the amino acid sequence as set forth in SEQ ID NO: 70, and
light chain CDR3 comprising the amino acid sequence as set forth in SEQ ID NO: 71; or
(O) the antibody satisfying the above (N), which has a heavy chain variable region, in which the amino acids at position 54 and 55 of the heavy chain according to Kabat definition are substituted with other amino acids.

6. An antibody that the amino acid sequences of CDRs (complementarity determining regions) 1 to 3 thereof satisfy either the following (P) or (Q), or an antigen-binding fragment thereof:
(P) an antibody having:
heavy chain CDR1 comprising the amino acid sequence as set forth in SEQ ID NO: 73,
heavy chain CDR2 comprising the amino acid sequence as set forth in SEQ ID NO: 74,
heavy chain CDR3 comprising the amino acid sequence as set forth in SEQ ID NO: 75,
light chain CDR1 comprising the amino acid sequence as set forth in SEQ ID NO: 77,
light chain CDR2 comprising the amino acid sequence as set forth in SEQ ID NO: 78, and
light chain CDR3 comprising the amino acid sequence as set forth in SEQ ID NO: 79; or
(Q) the antibody satisfying the above (P), which has heavy chain and light chain variable regions, in which the amino acids at position 54 and 55 of the heavy chain and the amino acid at position 33 of the light chain according to Kabat definition are substituted with other amino acids.

7. An antibody that the amino acid sequences of CDRs (complementarity determining regions) 1 to 3 thereof satisfy either the following (R) or (S), or an antigen-binding fragment thereof:
(R) an antibody having:
heavy chain CDR1 comprising the amino acid sequence as set forth in SEQ ID NO: 89,
heavy chain CDR2 comprising the amino acid sequence as set forth in SEQ ID NO: 90,
heavy chain CDR3 comprising the amino acid sequence as set forth in SEQ ID NO: 91,
light chain CDR1 comprising the amino acid sequence as set forth in SEQ ID NO: 93,
light chain CDR2 comprising the amino acid sequence as set forth in SEQ ID NO: 94, and
light chain CDR3 comprising the amino acid sequence as set forth in SEQ ID NO: 95; or
(S) the antibody satisfying the above (R), which has heavy chain and light chain variable regions, in which the amino acids at positions 52a and 53 of the heavy chain and the amino acid at position 33 of the light chain according to Kabat definition are substituted with other amino acids.

8. An antibody that the amino acid sequences of CDRs 1 to 3 of a heavy chain variable region thereof satisfy either following (T) or (U), and the amino acid sequences of CDRs 1 to 3 of a light chain variable region thereof satisfy any of the following (t) to (v), or an antigen-binding fragment thereof:
(T) an antibody having:
heavy chain CDR1 comprising the amino acid sequence as set forth in SEQ ID NO: 49,
heavy chain CDR2 comprising the amino acid sequence as set forth in SEQ ID NO: 50, and
heavy chain CDR3 comprising the amino acid sequence as set forth in SEQ ID NO: 113; or
(U) an antibody having:
heavy chain CDR1 comprising the amino acid sequence as set forth in SEQ ID NO: 183,
heavy chain CDR2 comprising the amino acid sequence as set forth in SEQ ID NO: 50, and
heavy chain CDR3 comprising the amino acid sequence as set forth in SEQ ID NO: 113; and
(t) an antibody having:
light chain CDR1 comprising the amino acid sequence as set forth in SEQ ID NO: 117,
light chain CDR2 comprising the amino acid sequence as set forth in SEQ ID NO: 54, and
light chain CDR3 comprising the amino acid sequence as set forth in SEQ ID NO: 55;
(u) an antibody having:
light chain CDR1 comprising the amino acid sequence as set forth in SEQ ID NO: 186,
light chain CDR2 comprising the amino acid sequence as set forth in SEQ ID NO: 54, and
light chain CDR3 comprising the amino acid sequence as set forth in SEQ ID NO: 55; and
(v) an antibody having:
light chain CDR1 comprising the amino acid sequence as set forth in SEQ ID NO: 188,
light chain CDR2 comprising the amino acid sequence as set forth in SEQ ID NO: 54, and
light chain CDR3 comprising the amino acid sequence as set forth in SEQ ID NO: 55.

9. An antibody that has:
heavy chain CDR1 comprising the amino acid sequence as set forth in SEQ ID NO: 73,
heavy chain CDR2 comprising the amino acid sequence as set forth in SEQ ID NO: 147,
heavy chain CDR3 comprising the amino acid sequence as set forth in SEQ ID NO: 75,
light chain CDR1 comprising the amino acid sequence as set forth in SEQ ID NO: 149,
light chain CDR2 comprising the amino acid sequence as set forth in SEQ ID NO: 78, and
light chain CDR3 comprising the amino acid sequence as set forth in SEQ ID NO: 79,
or an antigen-binding fragment thereof.

10. An antibody that has:
heavy chain CDR1 comprising the amino acid sequence as set forth in SEQ ID NO: 89,
heavy chain CDR2 comprising the amino acid sequence as set forth in SEQ ID NO: 151,
heavy chain CDR3 comprising the amino acid sequence as set forth in SEQ ID NO: 91,
light chain CDR1 comprising the amino acid sequence as set forth in SEQ ID NO: 93,
light chain CDR2 comprising the amino acid sequence as set forth in SEQ ID NO: 94, and
light chain CDR3 comprising the amino acid sequence as set forth in SEQ ID NO: 95,
or an antigen-binding fragment thereof.

11. An antibody that binds to CDCP1 and has low binding property to human CD34-positive cells, wherein the antibody competitively inhibits the binding of the antibody according to any one of claims 3 to 10, or an antigen-binding fragment thereof.

12. The antibody according to any one of claims 1 to 11, which is a humanized antibody, or the antigen-binding fragment thereof.

13. The antibody according to any one of claims 1 to 12, which binds to a substance having antitumor activity, or the antigen-binding fragment thereof.

14. The antibody according to claim 13, wherein the substance having antitumor activity is an anticancer agent, or the antigen-binding fragment thereof.

15. The antigen-binding fragment according to any one of claims 1 to 14, which is Fab, Fab', F(ab')₂, Fv, a single chain antibody, scFv, an scFv dimer, or dsFv.

16. A pharmaceutical composition, comprising the antibody according to any one of claims 1 to 15 or the antigen-binding fragment thereof.
